# EUROPEAN PATENT APPLICATION

(11) **EP 4 253 424 A1**
(43) Date of publication of application: **04.10.2023**
(21) Application number: 21897059.8
(22) Date of filing: 25.11.2021
(51) Int. Cl.: C07K 16/46, C12N 15/13, C12N 15/53, C12N 5/00, A61K 39/395

(54) **BISPECIFIC ANTIBODY AND USE THEREOF**

(30) Priority: 26.11.2020 CN 202011353447
(71) Applicant: Shanghai Huaota Biopharmaceutical Co., Ltd., Shanghai 201203 (CN); Huabo Biopharm (Shanghai) Co., Ltd., Shanghai 201203 (CN)
(72) Inventor: ZHU, Xiangyang, Shanghai 201203 (CN); CUI, Xiaopei, Shanghai 201203 (CN); XU, Jingen, Shanghai 201203 (CN); YU, Haijia, Shanghai 201203 (CN)
(74) Representative: Weickmann & Weickmann PartmbB
(86) International application number: PCT/CN2021/133003
(87) International publication number: WO 2022/111559

(57) **Abstract**

Provided in the present application is a multispecific antibody, which includes a first targeting moiety capable of specifically binding to the TIGIT protein and a second targeting moiety capable of binding to a tumor-associated antigen, and the multispecific antibody can be used to treat a tumor. Further provided in the present application is the use of the multispecific antibody as a drug.

## Description

### FIELD OF THE INTVENTION

The present application relates to the field of biomedicine, and in particular, relates to an anti-PD-L1/TIGIT bispecific antibody and uses thereof.

### BACKGROUND OF THE INVENTION

Depending on the cell characteristics of neoplasms and the degree of harm to organisms, tumors can be divided into two major categories, namely, benign tumors and malignant tumors. The malignant tumor disease is a major disease endangering human health in today's society, with a lethal degree ranking second. Conventional means for treating solid tumors includes surgery, chemotherapy, radiotherapy, molecularly targeted drug therapy, immunotherapy, etc. Many patients with solid tumors have already been in the advanced stage by the time when these solid tumors are found, and they have lost the opportunity for surgical treatment. Moreover, most patients have weak constitutions and are often unable to withstand the strong side effects caused by radiotherapy and chemotherapy treatment. Due to advantages of low toxicity and high efficacy, biological therapeutics, especially the cancer immunotherapies represented by immune checkpoint antibodies such as PD-1, have been increasingly widely used, and may be the only option in particular for patients with advanced solid tumors that are metastatic, recurrent, or refractory or on which existing standard chemotherapy methods are ineffective.

Most of the immune checkpoint antibody medicaments currently available on the market are monoclonal antibodies, most of which are against one specific target. However, drug resistance or no response may occur to patients receiving the monoclonal antibody therapy, and the in vivo influencing factors of many diseases are multifaceted, including different signaling pathways, different cytokines and receptor regulatory mechanisms, etc. The single-target immunotherapy is not sufficient to destroy tumor cells. Therefore, the development of polyspecific antibodies, for example bispecific antibodies (BsAb), may solve the problems of off-target toxicity, low response rate and drug tolerance of monoclonal antibody drugs, and expand the population benefited from the immunotherapy. Although the bispecific antibodies are a hot topic in the research and development of antibody medicaments, they face many challenges, such as preclinical evaluation models and problems such as low expression level, poor stability, complex process, and large difference in quality control. As a result, the research and development of bispecific antibodies has been beset with difficulties. Therefore, there is an urgent need to develop an antitumor medicament that is good in specificity and efficacy and easy to prepare.

### SUMMARY OF THE INVENTION

The present application provides a polyspecific antibody having one or more of the following properties: (1) capability of specifically binding to human PD-L1 and human TIGIT with high affinity, respectively; (2) capability of binding to a PD-L1 protein and a TIGIT protein at the same time; (3) capability of keeping a binding configuration of the polyspecific antibody and a bound target unchanged and maintaining molecular stability; (4) the purity of above 90% for the polyspecific antibody under the preliminary culture conditions; (5) capability of maintaining the binding affinity of the bound target, and exerting the synergistic effect of dual targets as compared a single anti-PD-L1 antibody (such as HB0023) and a single anti-TIGIT antibody (such as HB0030); and (6) capability of effectively killing tumor cells (especially tumors with high PD-L1 expression), for example, solid tumors. The preparation method for the polyspecific antibody of the present application is simple and feasible, and has a good application prospect.

In one aspect, the present application provides a polyspecific antibody, comprising a first targeting moiety capable of specifically binding to a TIGIT protein, wherein said first targeting moiety comprises an isolated antigen-binding protein, said isolated antigen-binding protein comprises a heavy-chain variable region VH, and said VH comprises HCDR1, HCDR2, and HCDR3, wherein said HCDR1 comprises an amino acid sequence as set forth in SEQ ID NO:3, said HCDR2 comprises an amino acid sequence as set forth in SEQ ID NO : 4 or 42, and said HCDR3 comprises an amino acid sequence as set forth in SEQ ID NO: 5 or 43.

In some embodiments, said polyspecific antibody comprises a light-chain variable region VL, and said VL comprises LCDR1, LCDR2, and LCDR3, wherein said LCDR1 comprises an amino acid sequence as set forth in SEQ ID NO: 6 or 52, said LCDR2 comprises an amino acid sequence an amino acid sequence as set forth in SEQ ID NO: 7, and said LCDR3 comprises an amino acid sequence as set forth in SEQ ID NO: 8 or 53.

In some embodiments, said polyspecific antibody comprises a heavy-chain variable region VH, wherein said VH comprises an amino acid sequence as set forth in any one of SEQ ID NOs: 110, 1, 9, 12, 14, and 32.

In some embodiments, said polyspecific antibody comprises a light-chain variable region VL, wherein said VL comprises an amino acid sequence as set forth in any one of SEQ ID NOs: 111,2, 10, 11, 13, 33, and 34.

In some embodiments, said polyspecific antibody further comprises a second targeting moiety.

In some embodiments, said second targeting moiety is capable of specifically binding to a tumor-associated antigen.

In some embodiments, second targeting moiety is capable of specifically binding to a PD-L1 protein. In some embodiments, said second targeting moiety comprises an antibody or an antigen-binding fragment, and said antibody capable of binding to said PD-L1 protein comprises HCDR1, HCDR2, and HCDR3, wherein said HCDR1, HCDR2, and HCDR3 sequentially comprise amino acid sequences as set forth in SEQ ID NO: 100, 101, and 102, respectively.

In some embodiments, said antibody capable of binding to said PD-L1 protein comprises LCDR1, LCDR2, and LCDR3, wherein said LCDR1, LCDR2, and LCDR3 sequentially comprise amino acid sequences as set forth in SEQ ID NO: 103, 104, and 105, respectively.

In some embodiments, said polyspecific antibody comprises a first polypeptide chain and a second polypeptide chain, wherein said first polypeptide chain comprises said heavy-chain variable region VH of said antibody capable of binding to said PD-L1 protein, said heavy-chain variable region VH capable of binding to said TIGIT protein, and said light-chain variable region VL capable of binding to said TIGIT protein; and said second polypeptide chain comprises said light-chain variable region VL of said antibody capable of binding to said PD-L1 protein; or, said first polypeptide chain comprises said heavy-chain variable region VH of said antibody capable of binding to said TIGIT protein, said heavy-chain variable region VH capable of binding to said PD-L1 protein, and said light-chain variable region VL capable of binding to said PD-L1 protein; and said second polypeptide chain comprises said light-chain variable region VL of said antibody capable of binding to said TIGIT protein;

In some embodiments, in said first polypeptide chain, said VH of said antibody capable of binding to said TIGIT protein and said VL of said antibody capable of binding to said TIGIT protein form scFv; or, in said first polypeptide chain, said VH of said antibody capable of binding to said PD-L 1 protein and said VL of said antibody capable of binding to said PD-L1 protein form scFv.

In some embodiments, said first polypeptide chain comprises an amino acid sequence as set forth in any one in the group consisting of: SEQ ID NOs: 78 to 83, and 85 to 87.

In some embodiments, said second polypeptide chain comprises an amino acid sequence as set forth in SEQ ID Nos: 84 or 88.

In another aspect, the present application provides an isolated nucleic acid molecule or isolated nucleic acid molecules, encoding said polyspecific protein.

In another aspect, the present application provides a vector comprising said nucleic acid molecule or nucleic acid molecules.

In another aspect, the present application provides a cell comprising said isolated nucleic acid molecule or isolated nucleic acid molecules or said vector as defined.

In another aspect, the present application provides a preparation method for said polyspecific antibody, comprising culturing said cell under such a condition that said polyspecific antibody is expressed.

In another aspect, the present application provides a pharmaceutical composition, comprising said isolated antibody, said nucleic acid molecule or nucleic acid molecules, said vector, and/or said cell, and optionally a pharmaceutically acceptable adjuvant.

In another aspect, the present application provides use of said polyspecific antibody, said nucleic acid molecule or nucleic acid molecules, and/or said pharmaceutical composition in the preparation of a medicament for preventing, relieving and/or treating diseases or disorders.

Other aspects and advantages of the present application may be readily perceived by those skilled in the art from the detailed description below. The detailed description below only illustrates and describes the exemplary embodiments of the present application. As would be appreciated by those skilled in the art, the content of the present application allows those killed in the art to change the specific embodiments disclosed without departing from the spirit and scope involved in the present application. Accordingly, the accompanying drawings and the description in the specification of the present application are merely for an exemplary but not restrictive purpose.

### BRIEF DESCRIPTION OF THE DRAWINGS

The specific features of the present invention involved in the present application are listed in the appended claims. The characteristics and advantages of the present invention involved in the present application may be better understood by referring to the exemplary embodiments and the accompanying drawings described in detail below. A brief description of the drawings is as follows:
FIG. 1 shows the binding activity of TIGIT chimeric antibodies to cells expressing human TIGIT;
FIG. 2 shows the binding activity of TIGIT humanized antibodies to cells expressing human TIGIT;
FIG. 3 shows the blocking activity of TIGIT chimeric antibodies against the binding of human TIGIT to its ligand CD155;
FIG. 4 shows the blocking activity of TIGIT humanized antibodies against the binding of human TIGIT to its ligand CD155;
FIG. 5 shows the efficacy test on TIGIT antibodies in animals;
FIG. 6 shows the efficacy test on TIGIT antibodies in animals versus a control antibody Tiragolumab;
FIG. 7Ato FIG. 7B show exemplary structures of the polyspecific antibodies described in the present application;
FIG. 8 shows the SDS-PAGE electrophoretogram results of the polyspecific antibody TIGIT-IgG-PDL1-scFv described in the present application;
FIG. 9 shows the SDS-PAGE electrophoretogram results of the polyspecific antibody PDL1-IgG-TIGIT-scFv described in the present application;
FIG. 10 shows the functional inhibition effect of the polyspecific antibody PDL 1- IgG- TIGIT-scFv described in the present application against PDL1;
FIG. 11 shows the functional inhibition effect of the polyspecific antibody TIGIT-IgG-PDL1-scFv described in the present application against TIGIT; and
FIG. 12 shows the therapeutic effect of the polyspecific antibody described in the present application on a mouse tumor model.

### DETAILED DESCRIPTION OF THE PREFERRED EMBODIMENTS

The embodiments of the invention of the present application will be illustrated by specific examples below. Those familiar with this technology can readily understand other advantages and effects of the present application from the content disclosed in the specification.

### TERMS & DEFINITIONS

In the present application, the term "TIGIT", referred to as "T cell immunoreceptor with Ig and ITIM domains", is generally a member of the poliovirus receptor (PVR) family of immunoglobulins. It can bind to PVR/CD155 and Nectin-2/CD112. The TIGIT in the present application may refer to the TIGIT protein from any vertebrate source, including mammals, such as primates (for example, humans, rhesus monkeys, and cynomolgus monkeys) and rodents (e.g., mice and rats). This term encompasses full-length TIGIT or fragments thereof (such as their mature fragments lacking a signal peptide), unprocessed TIGITs, any form of TIGIT due to processing in cells, and artificially synthesized TIGITs. This term also encompasses the variants of TIGIT, such as splice variants or allelic variants. In some cases, the TIGIT is human TIGIT, of which the amino acid sequence comprises an amino acid sequence as set forth in UniProt Accession No. Q495A1. In some cases, the TIGIT is cynomolgus monkey TIGIT, of which the amino acid sequence comprises an amino acid sequence as set forth in UniProt Accession No. G7NXM4. In some cases, the TIGIT is a mouse TIGIT, of which the amino acid sequence comprises an amino acid sequence as set forth in UniProt Accession No. P86176.

In the present application, the term "PD-L1" generally refers to a programmed cell death ligand 1, also referred to as B7 homolog 1, B7-H1, differentiated cluster 274, (3)274, or CD274. After binding to PD-1, it down-regulates T cell activation and cytokine secretion. "PD-L1" comprises any natural PD-L1 of any vertebrate origin, including mammals, such as primates (for example, humans and cynomolgus monkeys) and rodents (e.g., mice and rats). The terms described encompass "full-length" unprocessed PD-L1, and any form of PD-L1 produced from cell processing. PD-L1 can exist as a transmembrane protein or as a soluble protein. "PD-L1" comprises complete PD-L1 and fragments thereof, and further comprises functional variants, isoforms, species homologs, derivatives, analogues, and analogues having at least one common epitope with PD-L1. The basic structure of PD-L1 consists of four domains: an extracellular Ig-like V-type domain as well as an Ig-like C2-type domain, a transmembrane domain, and a cytoplasmic domain. Exemplary human PD-L1 amino acid sequences can be found under NCBI accession number NP_001254653 or UniProt accession number Q9NZQ7. In the present application, the term "antigen-binding protein" generally refers to a protein comprising a moiety that binds to an antigen, and optionally a scaffold or skeleton moiety that allows the moiety that binds to the antigen to adopt a conformation that promotes the binding of the antigen-binding protein to the antigen. Examples of the antigen-binding protein include, but are not limited to, antibodies, antigen-binding fragments (Fab, Fab', F(ab)₂, Fv fragments, F(ab')₂, scFv, di-scFv and/or dAb), immunoconjugates, multispecific antibodies (for example, bispecific antibodies), antibody fragments, antibody derivatives, antibody analogs, or fusion proteins, etc., as long as they exhibit the desired antigen-binding activity.

In the present application, the term "Fab" generally refers to a fragment comprising a heavy-chain variable domain and a light-chain variable domain, and also comprising a light-chain constant domain and a heavy-chain first constant domain (CH1). The term "Fab'" generally refers to a fragment that is different from Fab by the addition of a few residues (comprising one or more cysteines from the hinge region of an antibody) to a carboxyl terminus of the heavy-chain CH1. The term "F(ab')₂" generally refers to a dimer of Fab', comprising an antibody fragment in which two Fab fragments are linked by a disulfide bridge on the hinge region. The term "Fv" generally refers to the smallest antibody fragment that comprises a complete antigen recognition and binding site. In some cases, this fragment may consist of a dimer in which one heavy-chain variable region and one light-chain variable region are tightly non-covalently bound; and the term "dsFv" generally refers to a disulfide-stabilized Fv fragment, with a disulfide bond between a single light-chain variable region and a single heavy-chain variable region. The term "dAb fragment" generally refers to an antibody fragment consisting of a VH domain. In the present application, the term "scFv" generally refers to a monovalent molecule produced by covalently linking and pairing one heavy-chain variable domain with one light-chain variable domain of an antibody by means of a flexible peptide linker. Such scFv molecule may have the following general structure: NH₂-VL-linker-VH-COOH or NH₂-VH-linker-VL-COOH.

In the present application, the term "antibody" is used in the broadest sense, and specifically encompasses, but is not limited to, monoclonal antibodies (including full-length monoclonal antibodies containing two light chains and two heavy chains), polyclonal antibodies, multispecific antibodies (for example, bispecific antibodies), humanized antibodies, fully human antibodies, chimeric antibodies, and camelized single-domain antibodies. The "antibody" may generally comprise a protein in which at least two heavy chains (HC) and two light chains (LC) are linked to each other by disulfide bonds, or an antigen-binding fragment thereof. Each heavy chain comprises a heavy-chain variable region (VH) and a heavy-chain constant region. In some naturally occurring IgG, IgD, and IgA antibodies, a heavy-chain constant region comprises three domains, namely, CH1, CH2, and CH3. In some naturally occurring antibodies, each light chain comprises a light-chain variable region (VL) and a light-chain constant region. The light-chain constant region comprises one domain, i.e., CL. The regions VH and VL may be further subdivided into hyper-denatured regions, called complementarity determining regions (CDR), which alternate with conserved regions called framework regions (FR). Each VH or VL comprises three CDRs and four FRs, which are arranged in the following order from an amino terminus to a carboxyl terminus: FR1, CDR1, FR2, CDR2, FR3, CDR3, and FR4. The variable domains of the naturally occurring heavy and light chains each comprise four FRs (H-FR1, H-FR2, H-FR3, H-FR4, L-FR1, L-FR2, L-FR3, L-FR4), most of which have a β-pleated configuration and which are linked by three CDRs to form a loop linkage, and to form a portion of a β-pleated structure in some cases. The CDRs in each chain are closely brought together by the FR, and form, together with the CDRs from another chain, an antigen-binding site of the antibody. The constant region of the antibody can mediate the binding of immunoglobulins to host tissues or factors, including various cells (for example, effector cells) of the immune system and the first component (Clq) of the classical complement system.

In the present application, the term "variable" generally refers to the fact that some fractions of the sequence of the variable domain of an antibody change drastically, which contributes to the binding and specificity of various specific antibodies to their specific antigens. However, the variability is not uniformly distributed throughout the variable region of the antibody. It is concentrated in three segments, known as complementarity determining regions (CDRs) or hypervariable regions (HVRs), in the light-chain and heavy-chain variable regions. A more highly conserved moiety in the variable domain is called a framework (FR). In the art, the CDR of an antibody may be defined by a variety of methods, for example, the Kabat definition rules based on sequence variability (see, Kabat et al., Sequences of Proteins of Immunological Interest, Fifth Edition, National Institutes of Health, Besse Star, Maryland (1991)), the Chothia definition rules based on the location of the structural loop regions (see, A1-Lazikani et al., JMol Biol 273: 927-48, 1997), and the IMGT definition rules based on the concepts in IMGT-ONTOLOGY and IMGT Scientific chart rules.

IMGT refers to the ImMunoGeneTics information system, which is a global reference database for immunogenetics and immunoinformatics (http://www.imgt.org). IMGT specializes in immunoglobulins (IGs) or antibodies from humans and other vertebrates, T cell receptors (TRs), major histocompatibility (MH), and immunoglobulin superfamily (IgSF) from vertebrates and non-vertebrates, MH superfamily (MhSF), and immune system related proteins (RPIs).

In the present application, the term "Kabat antibody numbering scheme " generally refers to a numbering system for amino acid residues in the heavy-chain and light-chain variable regions of an antibody or an antigen-binding moiety (Kabat et al. (1971) Ann. NY Acad. Sci. 190: 382-391 and Kabat, E.A. et al. (1991), Sequence of Proteins of Immunological Interest, Fifth Edition, U.S. Department of Human Health Services, NIH Publication No. 91-3242). For the heavy-chain variable region, CDR1 refers to amino acids at positions 31 to 35 of the heavy-chain variable region, CDR2 refers to amino acids at positions 50 to 65 of the heavy-chain variable region, and CDR3 refers to amino acids at positions 95 to 102 of the heavy-chain variable region. For the light-chain variable region, CDR1 refers to amino acids at positions 24 to 34 of the light-chain variable region, CDR21 refers to amino acids at positions 50 to 56 in the light-chain variable region, and CDR3 refers to amino acids at positions 89 to 97.

In the present application, the term "isolated" antigen-binding protein generally refers to an antigen-binding protein that has been identified, isolated, and/or recovered from (for example, native or recombinant) components of the environment in which it is produced. Contaminant components of the environment in which it is produced are generally substances that interfere with its investigational, diagnostic or therapeutic use, and may include enzymes, hormones, and other proteinaceous or non-proteinaceous solutes. An isolated antigen-binding protein or an antibody is generally prepared by at least one purification step.

In the present application, the term "polyspecific antibody" generally refers to an antibody having a variable region recognizing more than one epitope on one or more antigens. The polyspecific antibodies include, but are not limited to, full-length antibodies, antibodies with two or more VL and VH domains, antibody fragments such as Fab, Fv, dsFv, and scFv, double antibodies, bispecific double and triple antibodies, and antibody fragments that have been covalently or non-covalently linked. In some embodiments, the polyspecific antibody may be a "bispecific antibody" that recognizes two different epitopes on the same or different antigens.

In the present application, the term "monoclonal antibody" generally refers to an antibody obtained from a group of substantially homogeneous antibodies, that is, a cluster in which several antibodies are the same, except for a few natural mutants that may exist. The monoclonal antibody is generally highly specific for a single antigen site. Moreover, unlike conventional polyclonal antibody preparations (which generally comprise different antibodies directed against different determinants), each monoclonal antibody is directed against a single determinant on the antigen. In addition to their specificity, the advantage of monoclonal antibodies lies in that they may be synthesized by hybridoma culture, without being contaminated by other immunoglobulins. The modifier "monoclonal" indicates the characteristics of an antibody obtained from a substantially homogeneous antibody population, and is not construed as requiring the production of the antibody by any specific method. For example, the monoclonal antibodies used in the present application may be prepared in hybridoma cells, or may be prepared by recombinant DNA methods.

In the present application, the term "chimeric antibody" generally refers to an antibody in which the variable region is derived from one species and the constant region is derived from another species. Generally, the variable region is derived from an antibody ("parent antibody") in an experimental animal such as a rodent, and the constant region is derived from a human antibody, such that the possibility of causing an adverse immune response in an individual human by the resulting chimeric antibody is reduced as compared with the parental (for example, mouse-derived) antibody.

In the present application, the term "humanized antibody" generally refers to an antibody in which some or all of the amino acids outside the CDR of a non-human antibody (such as a mouse antibody) have been replaced by corresponding amino acids derived from human immunoglobulins. In the CDR, small additions, deletions, insertions, substitutions, or modifications to the amino acids may also be allowed, as long as they still retain the capability of the antibody to bind to a specific antigen. The humanized antibody may optionally comprise at least a portion of a constant region of a human immunoglobulin. The "humanized antibody" reserves the antigen specificity similar to that of the original antibody. The "humanized" form of a non-human (for example, mouse) antibody may minimally comprise a chimeric antibody derived from a non-human immunoglobulin sequence. In some cases, CDR residues in a human immunoglobulin (receptor antibody) may be replaced with CDR residues from a non-human species (donor antibody) (such as a mouse, a rat, a rabbit, or a non-human primate) with the desired properties, affinity, and/or capability. In some cases, FR residues of the human immunoglobulin may be replaced with corresponding non-human residues. In addition, the humanized antibody may comprise an amino acid modification that is not present in the receptor antibody or in the donor antibody. These modifications may be made to further improve the properties such as binding affinity of the antibody.

In the present application, the term "fully human antibody" generally refers to an antibody that is obtained by transferring a human antibody-encoding gene into a genetically engineered antibody gene-deficient animal to allow the animal to express it. All moieties of the antibody (comprising the variable and constant regions of the antibody) are encoded by genes originating from humans. The fully human antibody can greatly reduce the side immune effects caused by heterologous antibodies on the human body. Methods for obtaining the fully human antibody in the art may include the phage display technology, the transgenic mouse technology, the ribosome display technology, the RNA-polypeptide technology, etc.

In the present application, the term "binding", "specific binding", or "specific to ..." generally refers to a measurable and reproducible interaction, such as the binding between an antigen and an antibody, whereby the existence of a target can be determined in the presence of a heterogeneous population of molecules (including biomolecules). For example, an antibody binds to an epitope by means of its antigen binding domain, and this binding requires some complementarity between the antigen binding domain and the epitope. For example, an antibody that specifically binds to a target (which may be an epitope) is an antibody that binds to the target with greater affinity, avidity, easiness, and/or duration than it binds to other targets. When an antibody binds to an epitope by means of its antigen binding domain more easily than its binding to a random, unrelated epitope, the antibody is referred to as "specifically binding" to that antigen. "Epitope" refers to a specific atomic group (for example, a sugar side chain, a phosphoryl group, a sulfonyl group) or amino acid, that binds to an antigen-binding protein (such as an antibody), on an antigen.

In the present application, the terms "KD" and "*K_{D}*" are used interchangeably and generally refer to the equilibrium dissociation constant. "KD" is the ratio of a dissociation rate constant (kdis, also known as "off-rate (koff)" or "kd") to an association rate constant (kon, also known as "on-rate (kon)" or "ka"). The association rate constant (ka), the dissociation rate constant (kd), and the equilibrium dissociation constant (KD) can used to represent the binding affinity of an antigen-binding protein (for example, an antibody) to an antigen. Methods for determining the association and dissociation rate constants are well known in the art, comprising but not limited to the biofilm interference technology (BLI), the radioimmunoassay (RIA), the equilibrium dialysis, the surface plasmon resonance (SPR), the fluorescence resonance energy transfer (FRET), the co-immunoprecipitation (Co-IP), and the protein chip technology. If the measurement is performed under different conditions (for example, in terms of salt concentration and pH), the measured affinity of a specific protein-protein interaction may be different.

In the present application, the term "reference antibody" generally refers to an antibody with which the antigen-binding protein described in the present application competes for binding to the antigen TIGIT.

In the present application, the term "ADCC" or "antibody-dependent cell-mediated cytotoxicity" generally refers to such a form of cytotoxicity that some secretory immunoglobulins bind to Fc receptors (FcR) on some cytotoxic effector cells (for example, NK cells, neutrophils, and macrophages), such that these cytotoxic effector cells can specifically bind to target cells carrying the antigens, and then kill the target cells using cytotoxins. Main cells (for example, NK cells) that mediate ADCC express FcyRIII only, while monocytes express FcyRI, FcγRII, and FeyRIII (see Ravetch and Kinet, Annu. Rev. Immunol. 9: 457-92 (1991), Page 464, Table 3). *In vitro* and/or *in vivo* cytotoxicity assays may be performed to assess the ADCC activity of the molecules of interest. For example, the *in vitro* ADCC assays may be performed. A reference may be made to the description in U.S. Patent No. 5,500,362 or No. 5,821,337 or U.S. Patent No. 6,737,056 (Presta). Effector cells available for such assays comprise PBMC and NK cells. Alternatively/additionally, the ADCC activity of the molecule of interest may be assessed *in vivo,* for example, in an animal model such as that disclosed in Clynes et al., PNAS (USA) 95:652-656 (1998). For example, an Fc receptor (FcR) binding assay may be performed to ensure that the antibody is short of FcyR binding (and thus may lack ADCC activity), but retains FcRn binding capability.

The ADCC activity may be reduced by modifying the Fc region. In some cases, sites affecting the binding to an Fc receptor may be removed. For example, sites that are not salvage receptor binding sites are removed. In some cases, the Fc region may be modified to remove ADCC sites. The ADCC sites are known in the art. For the ADCC sites of IgG1, see, for example, Sarmay et al.(1992) Molec. Immunol. 29 (5):633-9.

In the present application, the term "between" generally refers to that the C-terminus of an amino acid fragment is directly or indirectly linked to the N-terminus of a first amino acid fragment, and the N-terminus of the amino acid fragment is directly or indirectly linked to the C-terminus of a second amino acid fragment. In a light chain, for example, the N-terminus of the L-FR2 is directly or indirectly linked to the C-terminus of the LCDR1, and the C-terminus of the L-FR2 is directly or indirectly linked to the N-terminus of the LCDR2. For another example, the N-terminus of the L-FR3 is directly or indirectly linked to the C-terminus of the LCDR2, and the C-terminus of the L-FR3 is directly or indirectly linked to the N-terminus of the LCDR3. In a heavy chain, for example, the N-terminus of the H-FR2 is directly or indirectly linked to the C-terminus of the HCDR1, and the C-terminus of the H-FR2 is directly or indirectly linked to the N-terminus of the HCDR2. For another example, the N-terminus of the H-FR3 is directly or indirectly linked to the C-terminus of the HCDR2, and the C-terminus of the H-FR3 is directly or indirectly linked to the N-terminus of the HCDR3. In the present application, the "first amino acid fragment" and the "second amino acid fragment" may be any amino acid fragments that are the same or different.

In the present application, the term "isolated" antigen-binding protein generally refers to an antigen-binding protein that has been identified, isolated, and/or recovered from (for example, native or recombinant) components of the environment in which it is produced. Contaminant components of the environment in which it is produced are generally substances that interfere with its investigational, diagnostic or therapeutic use, and may include enzymes, hormones, and other proteinaceous or non-proteinaceous solutes. An isolated antigen-binding protein or an antibody is generally prepared by at least one purification step. The isolated antigen-binding protein of the present application generally does not bind to antigens that are non-TIGIT antigens.

In the present application, the term "isolated nucleic acid molecule" or "isolated polynucleotide" generally refers to a genome, an mRNA, a cDNA, or a synthetic-origin DNA or RNA or a certain combination thereof. It is not associated with the all or some of polynucleotides found in nature, or is linked to polynucleotides to which it is not linked in nature.

In the present application, the term "vector" generally refers to a nucleic acid molecule capable of self-replication in a suitable host. It transfers an inserted nucleic acid molecule into and/or between host cells. The vector may include a vector mainly for inserting DNA or RNA into cells, a vector mainly for replicating DNA or RNA, and a vector mainly for expressing DNA or RNA transcription and/or translation. The vector also includes a carrier having a variety of the functions defined above. The vector may be a polynucleotide that may be transcribed and translated into a polypeptide when introduced into a suitable host cell. Generally, the vector may produce a desired expression product by culturing a suitable host cell containing the vector.

In the present application, the term "cell" generally refers to an individual cell, a cell line or a cell culture, which may contain or already contains a plasmid or vector comprising a nucleic acid molecule of the present application, or which is capable of expressing the antibody or antigen-binding fragment thereof in the present application. The cell may include a progeny of a single host cell. Due to natural, accidental or deliberate mutations, progeny cells and original parent cells may not be necessarily identical in terms of morphology or genome, as long as they are capable of expressing the antibody or antigen-binding fragment thereof in the present application. The cells may be obtained by transfecting cells *in vitro* using the vector of the present application. The cells may be prokaryotic cells (for example, *Escherichia coli*), or eukaryotic cells (for example, yeast cells, for example, COS cells, Chinese hamster ovary (CHO) cells, HeLa cells, HEK293 cells, COS-1 cells, NS0 cells, or myeloma cells). In some cases, the cells may be mammalian cells. For example, the mammalian cells may be CHO-K1 cells. In the present application, the term "recombinant cell" generally refers to a cell into which a recombinant expression vector is introduced. A recombinant host cell comprises not only certain specific cells, but also the progenies of these cells.

In the present application, the term "pharmaceutically acceptable adjuvant" generally comprises pharmaceutically acceptable carriers, excipients, or stabilizers, which are nontoxic for the cells or mammals that are exposed to them at the dose and concentration used. Generally, the physiologically acceptable carrier is a PH buffered aqueous solution. Examples of the physiologically acceptable carrier may comprise: buffers, such as phosphate, citrate, and other organic acids; antioxidants, including ascorbic acid; low-molecular-weight (less than about 10 residues) polypeptides, and proteins, such as serum albumin, gelatin, or immunoglobulin; hydrophilic polymers, such as polyvinylpyrrolidone; amino acids, such as glycine, glutamine, asparagine, arginine, or lysine; monosaccharides, disaccharides, and other carbohydrates, including glucose, mannose, or dextrin; chelating agents, such as EDTA; sugar alcohols, such as mannitol or sorbitol; salt-forming counterions, such as sodium; and/or nonionic surfactants, such as TWEEN^{™}, polyethylene glycol (PEG), and PLURONICS^{™}.

As used herein, the terms "administration" and "treatment" refer to the application of exogenous medicaments, therapeutic agents, diagnostic agents, or compositions to animals, humans, subjects, cells, tissues, organs, or biological fluids. "Administration" and "treatment" may refer to treatment, pharmacokinetics, diagnosis, studies, and experimental methods. The treatment of cells comprises contact between reagents and cells, contact between reagents and fluids, and contact between fluids and cells. "Administration" and "treatment" also mean *in vitro* and *ex vivo* treatment by means of reagents, diagnostics, and binding compositions, or by another cell. When applied to humans, animals, or research subjects, "treatment" refers to therapeutic treatment, prevention or preventive measures, study, and diagnosis. It encompasses the contact between TIGIT conjugates and humans or animals, subjects, cells, tissues, physiological compartments, or physiological fluids.

As used herein, the term "therapy" refers to the administration of an internal or external therapeutic agent, comprising any one of the TIGIT antigen-binding proteins and compositions thereof in the present application, to a patient who has one or more disease symptoms, and furthermore, the therapeutic agent is known to show a therapeutic effect against these symptoms. Generally, therapeutic agent is administered to the patient at an amount (therapeutically effective amount) for effectively alleviating one or more disease symptoms. The desired therapeutic effect comprises reducing the rate of disease progression, ameliorating or alleviating the disease state, and regressing or improving the prognosis. For example, if one or more symptoms related to a cancer are alleviated or eliminated in terms of, including but not limited to, reducing (or destroying) the proliferation of cancer cells, reducing symptoms derived from the disease, improving the quality of life for those individuals suffering from the disease, reducing the doses of other medicaments required for treating the disease, delaying the progression of the disease, and/or prolonging the survival of the individual, then the individual is deemed as being successfully "treated".

In the present application, the term "tumor" refers to all neoplastic cell growth and proliferation, both malignant and benign, as well as all pre-cancerous and cancerous cells and tissues.

As used herein, the term "optional" or "optionally" means that the event or situation described subsequently may occur but does not have to occur.

In the present application, the term "comprise" generally refers to the meaning of including, inclusive, containing, or encompassing. In some cases, it also means "is/are" and "consist of.

In the present application, the term "approximately" generally refers to a variation within a range of 0.5%-10% above or below a specified value, for example, a variation within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, and 10% above or below a specified value.

### DETAILED DESCRIPTION OF THE INVENTION

### TIGIT antigen-binding protein

In one aspect, the present application provides an isolated antigen-binding protein, which may comprise at least one CDR in a heavy-chain variable region VH. The VH may comprise an amino acid sequence as set forth in SEQ ID NO: 55.

In one aspect, the present application provides an isolated antigen-binding protein, which may comprise at least one CDR in a heavy-chain variable region VH. The VH may comprise an amino acid sequence as set forth in SEQ ID NO: 54.

In the present application, the VH of the antigen-binding protein may comprise HCDR1, HCDR2, and HCDR3.

The antigen-binding protein of the present application may comprise HCDR1, wherein the HCDR1 may comprise an amino acid sequence as set forth in SEQ ID NO: 3: GYSITSDYA. For example, this sequence may be a sequence determined according to IMGT definition rules.

The antigen-binding protein of the present application may comprise HCDR2, wherein the HCDR2 may comprise an amino acid sequence as set forth in SEQ ID NO: 56: IX₂X₃SGX₆X₇, in which X₂ is S or T, X₃ is S or Y, X₆ is A or S, and X₇ is P or T. For example, this sequence may be a sequence determined according to IMGT definition rules.

For example, the HCDR2 may comprise an amino acid sequence as set forth in any one of SEQ ID NOs: 4 and 42.

The antigen-binding protein of the present application may comprise HCDR2, wherein the HCDR2 may comprise an amino acid sequence as set forth in SEQ ID NO: 4: ITSSGST. For example, this sequence may be a sequence determined according to IMGT definition rules.

The antigen-binding protein of the present application may comprise HCDR3, wherein the HCDR3 may comprise an amino acid sequence as set forth in SEQ ID NO: 57: AX₂LX₄X₅X₆X₇YX₉X₁₀AMDY, in which X₂ is R or S, X₄ is D or G, X₅ is F or T, X₆ is D or G, X₇ is Nor Y, X₉ is G or absent, and X₁₀ is G or absent. For example, this sequence may be a sequence determined according to IMGT definition rules.

For example, the HCDR3 may comprise an amino acid sequence as set forth in any one of SEQ ID NOs: 5 and 43.

The antigen-binding protein of the present application may comprise HCDR3, wherein the HCDR3 may comprise an amino acid sequence as set forth in SEQ ID NO: 5: ARLDFGNYGGAMDY. For example, this sequence may be a sequence determined according to IMGT definition rules.

The antigen-binding protein of the present application may further comprise framework regions H-FR1, H-FR2, H-FR3, and H-FR4.

The antigen-binding protein of the present application may comprise a framework region H-FR1, wherein the H-FR1 may comprise an amino acid sequence as set forth in SEQ ID NO: 58: X₁VQLQESGPGLVKPSX₁₆X₁₇LSLTCTVX₂₅, in which X₁ is D or Q, X₁₆ is E or Q, X₁₇ is S or T, and X₂₅ is S or T. For example, this sequence may be a sequence determined according to IMGT definition rules.

For example, the H-FR1 may comprise an amino acid sequence as set forth in any one of SEQ ID NOs: 23 and 35.

The antigen-binding protein of the present application may comprise a framework region H-FR1, wherein the H-FR1 may comprise an amino acid sequence as set forth in SEQ ID NO: 23: QVQLQESGPGLVKPSETLSLTCTVS. For example, this sequence may be a sequence determined according to IMGT definition rules.

The antigen-binding protein of the present application may comprise a framework region H-FR2, wherein the H-FR2 may comprise an amino acid sequence as set forth in SEQ ID NO: 60: WX₂WIRQX₇PGX₁₀X₁₁X₁₂EWX₁₅GY, in which X₂ is I or N, X₇ is F or P, X₁₀ is K or N, X₁₁ is G, R, or K, X₁₂ is L or V, and X₁₅ is I or M. For example, this sequence may be a sequence determined according to IMGT definition rules.

For example, the H-FR2 may comprise an amino acid sequence as set forth in any one of SEQ ID NOs: 24 to 26 and 36 to 37.

The antigen-binding protein of the present application may comprise a framework region H-FR2, wherein the H-FR2 may comprise an amino acid sequence as set forth in SEQ ID NO: 59: WIWIRQPPGX₁₀X₁₁LEWIGY, in which X₁₀ is K or N, and X₁₁ is G or K. For example, this sequence may be a sequence determined according to IMGT definition rules.

For example, the H-FR2 may comprise an amino acid sequence as set forth in any one of SEQ ID NOs: 24 to 26.

The antigen-binding protein of the present application may comprise a framework region H-FR3, wherein the H-FR3 may comprise an amino acid sequence as set forth in SEQ ID NO: 61: X₁YNPSLKSRX₁₀X₁₁X₁₂X₁₃X₁₄DTSKNQFX₂₂LX₂₄LX₂₆X₂₇VTX₃₀X₃₁DTATYYC, in which X₁ is R, S, or Y, X₁₀ is I or V, X₁₁ is S or T, X₁₂ is F or I, X₁₃ is S or T, X₁₄ is R or V, X₂₂ is F or S, X₂₄ is K or Q, X₂₆ is S or T, X₂₇ is F or S , X₃₀ is A or T, and X₃₁ is A or E. For example, this sequence may be a sequence determined according to IMGT definition rules.

For example, the H-FR3 may comprise an amino acid sequence as set forth in any one of SEQ ID NOs: 27, 38, and 39.

The antigen-binding protein of the present application may comprise a framework region H-FR3, wherein the H-FR3 may comprise an amino acid sequence as set forth in SEQ ID NO: 27: YYNPSLKSRVTFSVDTSKNQFSLKLSSVTAADTATYYC. For example, this sequence may be a sequence determined according to IMGT definition rules.

The antigen-binding protein of the present application may comprise a framework region H-FR4, wherein the H-FR4 may comprise an amino acid sequence as set forth in SEQ ID NO: 62: WGQGTX₆VX₈VSS, in which X₆ is L or S, and X₈ is I or T. For example, this sequence may be a sequence determined according to IMGT definition rules.

For example, the H-FR4 may comprise an amino acid sequence as set forth in any one of SEQ ID NOs: 28, 40, and 41.

The antigen-binding protein of the present application may comprise a framework region H-FR4, wherein the H-FR4 may comprise an amino acid sequence as set forth in SEQ ID NO: 28: WGQGTLVTVSS. For example, this sequence may be a sequence determined according to IMGT definition rules.

The antigen-binding protein of the present application may comprise a heavy-chain variable region VH, wherein the VH may comprise an amino acid sequence as set forth in SEQ ID NO:55: X₁VQLQESGPGLVKPSX₁₆X₁₇LSLTCTVX₂₅GYSITSDYAWX₃₆WIRQX₄₁PGX₄₄X₄₅X₄₆EWX₄₉GY IX₅₃X₅₄SGX₅₇X₅₈X₅₉YNPSLKSRX₆₈X₆₉X₇₀X₇₁X₇₂DTSKNQFX₈₀LX₈₂LX₈₄X₈₅VTX₈₈X₈₉DTATYY CAX₉₈LX₁₀₀X₁₀₁X₁₀₂X₁₀₃YX₁₀₅X₁₀₆AMDYWGQGTX₁₁₆VX₁₁₈VSS, in which X ₁ is D or Q, X₁₆ is E or Q, X₁₇ is S or T, X₂₅ is S or T, X₃₆ is I or N, X₄₁ is F or P, X₄₄ is N or K, X₄₅ is G, K or R, X₄₆ is L or V, X₄₉ is I or M, X₅₃ is S or T, X₅₄ is S or Y, X₅₇ is A or S, X₅₈ is P or T, X₅₉ is R, S or Y, X₆₈ is I or V, X₆₉ is S or T, X₇₀ is F or I, X₇₁ is S or T, X₇₂ is R or V, X₈₀ is F or S, X₈₂ is K or Q, X₈₄ is S or T, X₈₅ is F or S, X₈₈ is A or T, X₈₉ is A or E, X₉₈ is R or S, X₁₀₀ is D or G, X₁₀₁ is F or T, X₁₀₂ is D or G, X₁₀₃ is N or Y, X₁₀₅ is G or absent, X₁₀₆ is G or absent, X₁₁₆ is L or S, and X₁₁₈ is I or T. For example, this sequence may be a sequence determined according to IMGT definition rules.

For example, the antigen-binding protein may comprise a heavy-chain variable region VH, wherein the VH may comprise an amino acid sequence as set forth in any one of SEQ ID NOs: 1, 9, 12, 14, and 32.

The isolated antigen-binding protein of the present application may comprise heavy-chain variable region VH, wherein the VH may comprise an amino acid sequence as set forth in SEQ ID NO: 54: QVQLQESGPGLVKPSETLSLTCTVSGYSITSDYAWIWIRQPPGX₄₄X₄₅LEWIGYITSSGSTYYN PSLKSRVTFSVDTSKNQFSLKLSSVTAADTATYYCARLDFGNYGGAMDYWGQGTLVTVSS, in which X ₄₄ is K or N, and X₄₅ is G or K. For example, this sequence may be a sequence determined according to IMGT definition rules.

For example, the antigen-binding protein may comprise a heavy-chain variable region VH, wherein the VH may comprise an amino acid sequence as set forth in any one of SEQ ID NOs: 9, 12, and 14. The antigen-binding protein of the present application may comprise a heavy-chain constant region, which may be derived from a human IgG, for example, human IgG1. In some cases, the Fc region of the human IgG1 may be modified to achieve the desired property (for example, ADCC KO). The modification may be an amino acid mutation. In some cases, the modification to the IgG1 may be L234A/L235A. That is, based on EU numbering, amino acids at positions 234 and 235 are mutated from leucine (L) to alanine (A), respectively. In some cases, the heavy-chain constant region of the antigen-binding protein of the present application may be derived from wild-type human IgG1.

For example, the antigen-binding protein of the present application may comprise a heavy-chain constant region, which may comprise an amino acid sequence as set forth in any one of SEQ ID NOs: 35 to 36.

The isolated antigen-binding protein of the present application may comprise at least one CDR in a light-chain variable region VL. The VL may comprise an amino acid sequence as set forth in SEQ ID NO: 64.

The isolated antigen-binding protein of the present application may comprise at least one CDR in a light-chain variable region VL. The VL may comprise an amino acid sequence as set forth in SEQ ID NO: 63.

In the present application, the VL of the antigen-binding protein may comprise LCDR1, LCDR2, and LCDR3.

The antigen-binding protein of the present application may comprise LCDR1, wherein the LCDR1 may comprise an amino acid sequence as set forth in SEQ ID NO: 65: QHVSX₅A, in which X₅ is N or T. For example, this sequence may be a sequence determined according to IMGT definition rules. For example, the LCDR1 may comprise an amino acid sequence as set forth in any one of SEQ ID NOs: 6 and 52.

The antigen-binding protein of the present application may comprise LCDR1, wherein the LCDR1 may comprise an amino acid sequence as set forth in SEQ ID NO: 6: QHVSTA. For example, this sequence may be a sequence determined according to IMGT definition rules.

The antigen-binding protein of the present application may comprise LCDR2, wherein the LCDR2 may comprise an amino acid sequence as set forth in SEQ ID NO: 7: SAS. For example, this sequence may be a sequence determined according to IMGT definition rules.

The antigen-binding protein of the present application may comprise LCDR3, wherein the LCDR3 may comprise an amino acid sequence as set forth in SEQ ID NO: 66: QQX₃YX₅X₆PX₈T, in which X₃ is H or Y, X₅ is I or S, X₆ is L or T, and X₈ is W or Y For example, this sequence may be a sequence determined according to IMGT definition rules.

For example, the LCDR3 may comprise an amino acid sequence as set forth in any one of SEQ ID NOs: 8 and 53.

The antigen-binding protein of the present application may comprise LCDR3, wherein the LCDR3 may comprise an amino acid sequence as set forth in SEQ ID NO: 8: QQHYITPYT. For example, this sequence may be a sequence determined according to IMGT definition rules.

The antigen-binding protein of the present application may further comprise framework regions L-FR1, L-FR2, L-FR3, and L-FR4.

The antigen-binding protein of the present application may comprise a framework region L-FR1, wherein the L-FR1 may comprise an amino acid sequence as set forth in SEQ ID NO: 68: DIX₃MTQSX₈X₉X₁₀X₁₁X₁₂X₁₃SX₁₅GDRVX₂₀ITCX₂₄AS, in which X₃ is Q or V, X₈ is H or P, X₉ is K or S, X₁₀ is F or S, X₁₁ is L or M, X₁₂ is F or S, X₁₃ is A or T, X₁₅ is I or V, X₂₀ is SorT, and X₂₄ is K or R. For example, this sequence may be a sequence determined according to IMGT definition rules. For example, the L-FR1 may comprise an amino acid sequence as set forth in any one of SEQ ID NOs: 15, 16, 44, and 45.

The antigen-binding protein of the present application may comprise a framework region L-FR1, wherein the L-FR1 may comprise an amino acid sequence as set forth in SEQ ID NO: 67: DIQMTQSPSSLSASVGDRVTITCX₂₄AS, in which X₂₄ is K or R. For example, this sequence may be a sequence determined according to IMGT definition rules.

For example, the L-FR1 may comprise an amino acid sequence as set forth in any one of SEQ ID NOs: 15 and 16.

The antigen-binding protein of the present application may comprise a framework region L-FR2, wherein the L-FR2 may comprise an amino acid sequence as set forth in SEQ ID NO: 70: ₁X₂WYQQKPGX₁₀X₁₁PKLLIX₁₇, in which X₁ is L or V, X₂ is A or N, X₁₀ is K or Q, X₁₁ is A or S, and X₁₇ is H or Y For example, this sequence may be a sequence determined according to IMGT definition rules.

For example, the L-FR2 may comprise an amino acid sequence as set forth in any one of SEQ ID NOs: 17, 18, 46, and 47.

The antigen-binding protein of the present application may comprise a framework region L-FR2, wherein the L-FR2 may comprise an amino acid sequence as set forth in SEQ ID NO: 69: X₁X₂WYQQKPGKAPKLLIY, in which X₁ is L or V, and X₂ is A or N. For example, this sequence may be a sequence determined according to IMGT definition rules.

For example, the L-FR2 may comprise an amino acid sequence as set forth in any one of SEQ ID NOs: 17 and 18.

The isolated antigen-binding protein of the present application may comprise a framework region L-FR3, wherein the L-FR3 may comprise an amino acid sequence as set forth in SEQ ID NO:72: YX₂X₃X₄GVPX8RFX₁₁GX₁₃X₁₄SGTDFTX₂₁TIX₂₄SX₂₆QX₂₈EDX₃₁AX₃₃YYC; in which X₂ is L or R, X₃ is Q or Y, X₄ is SorT, X₈ is D or S, X₁₁ is I, SorT, X₁₃ is R or S, X₁₄ is G or R, X₂₁ is F or L, X₂₄ is N or S, X₂₆ is L or V, X₂₈ is A or P, X₃₃ is F or L, and X₁₄ is T or V. For example, this sequence may be a sequence determined according to IMGT definition rules.

For example, the L-FR3 may comprise an amino acid sequence as set forth in any one of SEQ ID NOs: 19 to 21 and 48 to 50.

The antigen-binding protein of the present application may comprise a framework region L-FR3, wherein the L-FR3 may comprise an amino acid sequence as set forth in SEQ ID NO: 71: YX₂X₃SGVPSRFSGSX₁₄SGTDFTLTISSLQPEDFATYYC, in which X₂ is L or R, X₃ is Q or Y, and X₁₄ is G or R. For example, this sequence may be a sequence determined according to IMGT definition rules.

For example, the L-FR3 may comprise an amino acid sequence as set forth in any one of SEQ ID NOs: 19 to 21.

The antigen-binding protein of the present application may comprise a framework region L-FR4, wherein the L-FR4 may comprise an amino acid sequence as set forth in SEQ ID NO: 73: FGX₃GTKLEIK, in which X₃ is G or Q. For example, this sequence may be a sequence determined according to IMGT definition rules.

For example, the L-FR4 may comprise an amino acid sequence as set forth in any one of SEQ ID NOs: 22 and 51.

The antigen-binding protein of the present application may comprise a framework region L-FR4, wherein the L-FR4 may comprise an amino acid sequence as set forth in SEQ ID NO: 22: FGQGTKLEIK. For example, this sequence may be a sequence determined according to IMGT definition rules.

The isolated antigen-binding protein of the present application may comprise a light-chain variable region VL, wherein the VL may comprise an amino acid sequence as set forth in SEQ ID NO: 64: DIX₃MTQSX₈X₉X₁₀X₁₁X₁₂X1₃SX₁₅GDRVX₂₀ITCX₂₄ASQHVSX₃₁AX₃₃X₃₄WYQQKPGX₄₂X₄₃PK LLIX₄₉SASYX₅₄X₅₅X₅₆GVPX₆₀RFX₆₃GX₆₅X₆₆SGTDFTX₇₃TIX₇₆SX₇₈QX₈₀EDX₈₃AX₈₅YYCQQX_{9 1}YX₉₃X₉₄PX₉₆TFGX₁₀₀GTKLEIK, in which X₃ is Q or V, X₈ is H or P, X₉ is K or S, X₁₀ is F or S, X₁₁ is L or M, X₁₂ is F or S, X₁₃ is A or T, X₁₅ is I or V, X₂₀ is S or T, X₂₄ is K or R, X₃₁ is N or T, X₃₃ is L or V, X₃₄ is A or N, X₄₂ is K or Q, X₄₃ is A or S, X₄₉ is H or Y, X₅₄ is L or R, X₅₅ is Q or Y, X₅₆ is S or T, X₆₀ is D or S, X₆₃ is I, S or T, X₆₅ is S or R, X₆₆ is G or R, X₇₃ is L or F, X₇₆ is S or N, X₇₈ is L or V, X₈₀ is A or P, X₈₃ is F or L, X₈₅ is T or V, X₉₁ is H or Y, X₉₃ is I or S, X₉₄ is L or T, X₉₆ is W or Y, and X₁₀₀ is Q or G. For example, this sequence may be a sequence determined according to IMGT definition rules.

For example, the antigen-binding protein may comprise a light-chain variable region VL, wherein the VL may comprise an amino acid sequence as set forth in any one of SEQ ID NOs: 2, 10, 11, 13, 33, and 34.

The isolated antigen-binding protein of the present application may comprise light-chain variable region VL, wherein the VL may comprise an amino acid sequence as set forth in SEQ ID NO: 63: DIQMTQSPSSLSASVGDRVTITCX₂₄ASQHVSTAX₃₃X₃₄WYQQKPGKAPKLLIYSASYX₅₄X₅₅S GVPSRFSGSX₆₆SGTDFTLTISSLQPEDFATYYCQQHYITPYTFGQGTKLEIK, in which X₂₄ is K or R, X₃₃ is L or V, X₃₄ is A or N, X₅₄ is L or R, X₅₅ is Q or Y, and X₆₆ is G or R. For example, this sequence may be a sequence determined according to IMGT definition rules.

For example, the antigen-binding protein may comprise a light-chain variable region VL, wherein the VL may comprise an amino acid sequence as set forth in any one of SEQ ID NOs: 10, 11, and 13.

The antigen-binding protein of the present application may comprise a light-chain constant region, which may comprises a human light-chain constant region sequence, for example, a human κ light-chain constant region. For example, the light-chain constant region of the isolated antigen-binding protein of the present application may comprise an amino acid sequence as set forth in any one of SEQ ID NO: 31.

In the present application, the isolated antigen-binding protein may comprise antibody heavy-chain variable regions CDRs, namely, HCDR1, HCDR2, and HCDR3. The HCDR1 may comprise an amino acid sequence as set forth in SEQ ID NO: 3, the HCDR2 may comprise an amino acid sequence as set forth in SEQ ID NO: 56, and the HCDR3 may comprise an amino acid sequence as set forth in SEQ ID NO: 57.

For example, in the present application, the isolated antigen-binding protein may comprise antibody heavy-chain variable regions CDRs, namely, HCDR1, HCDR2, and HCDR3. The HCDR1 may comprise an amino acid sequence as set forth in SEQ ID NO: 3, the HCDR2 may comprise an amino acid sequence as set forth in any one of SEQ ID NOs: 4 and 42, and the HCDR3 may comprise an amino acid sequence as set forth in any one of SEQ ID NOs: 5 and 43.

In the present application, the isolated antigen-binding protein may comprise antibody heavy-chain variable regions CDRs, namely, HCDR1, HCDR2, and HCDR3. The HCDR1, the HCDR2, and the HCDR3 may sequentially comprise amino acid sequences as set forth in SEQ ID NO: 3, SEQ ID NO: 4, and SEQ ID NO: 5, respectively.

In the present application, the isolated antigen-binding protein may comprise antibody light-chain variable regions CDRs, namely, LCDR1, LCDR2, and LCDR3. The LCDR1 may comprise an amino acid sequence as set forth in SEQ ID NO: 65, the LCDR2 may comprise an amino acid sequence as set forth in SEQ ID NO: 7, and the LCDR3 may comprise an amino acid sequence as set forth in SEQ ID NO: 66.

For example, in the present application, the isolated antigen-binding protein may comprise antibody light-chain variable regions CDRs, namely, LCDR1, LCDR2, and LCDR3. The LCDR1 may comprise an amino acid sequence as set forth in any one of SEQ ID NOs: 6 and 52, the LCDR2 may comprise an amino acid sequence as set forth in SEQ ID NO: 7, and the HCDR3 may comprise an amino acid sequence as set forth in any one of SEQ ID NOs: 8 and 53.

In the present application, the isolated antigen-binding protein may comprise antibody light-chain variable regions CDRs, namely, LCDR1, LCDR2, and LCDR3. The LCDR1, the LCDR2, and the LCDR3 may sequentially comprise amino acid sequences as set forth in SEQ ID NO: 6, SEQ ID NO: 7, and SEQ ID NO: 8, respectively.

In the present application, the isolated antigen-binding protein may comprise HCDR1, HCDR2, HCDR3, LCDR1, LCDR2, and LCDR3. The HCDR1 may comprise an amino acid sequence as set forth in SEQ ID NO: 3; the HCDR2 may comprise an amino acid sequence as set forth in SEQ ID NO: 56; the HCDR3 may comprise an amino acid sequence as set forth in SEQ ID NO: 57; the LCDR1 may comprise an amino acid sequence as set forth in SEQ ID NO: 65; the LCDR2 may comprise an amino acid sequence as set forth in SEQ ID NO: 7; and the LCDR3 may comprise an amino acid sequence as set forth in SEQ ID NO: 66.

For example, in the present application, the isolated antigen-binding protein may comprise HCDR1, HCDR2, HCDR3, LCDR1, LCDR2, and LCDR3. The HCDR1 may comprise an amino acid sequence as set forth in SEQ ID NO: 3; the HCDR2 may comprise an amino acid sequence as set forth in any one of SEQ ID Nos: 4 and 42; the HCDR3 may comprise an amino acid sequence as set forth in any one of SEQ ID NOs: 5 and 43; the LCDR1 may comprise an amino acid sequence as set forth in any one of SEQ ID NOs: 6 and 52; the LCDR2 may comprise an amino acid sequence as set forth in SEQ ID NO: 7; and the LCDR3 may comprise an amino acid sequence as set forth in any one of SEQ ID NOs: 8 and 53.

In the present application, the isolated antigen-binding protein may comprise HCDR1, HCDR2, HCDR3, LCDR1, LCDR2, and LCDR3. The HCDR1, the HCDR2, the HCDR3, the LCDR1, the LCDR2, and the LCDR3 may sequentially comprise amino acid sequences as set forth in SEQ ID NO: 3, SEQ ID NO: 4, SEQ ID NO: 5, SEQ ID NO: 6, SEQ ID NO: 7, and SEQ ID NO: 8, respectively. In some cases, the isolated antigen-binding protein of the present application may comprise HCDR1, HCDR2, HCDR3, LCDR1, LCDR2, and LCDR3. The HCDR1, the HCDR2, the HCDR3, the LCDR1, the LCDR2, and the LCDR3 may sequentially comprise amino acid sequences as set forth in SEQ ID NO: 3, SEQ ID NO: 42, SEQ ID NO: 43, SEQ ID NO: 52, SEQ ID NO: 7, and SEQ ID NO: 53, respectively.

In the present application, the isolated antigen-binding protein may comprise a heavy-chain variable region VH and a light-chain variable region VL. The VH may comprise an amino acid sequence as set forth in SEQ ID NO: 55, and the VL may comprise an amino acid sequence as set forth in SEQ ID NO: 64.

In some cases, the isolated antigen-binding protein may comprise a heavy-chain variable region VH and a light-chain variable region VL. The VH may comprise an amino acid sequence as set forth in any one of SEQ ID NO:110, SEQ ID NO: 1, SEQ ID NO: 9, SEQ ID NO: 12, SEQ ID NO: 32, and SEQ ID NO: 14, and the VL may comprise an amino acid sequence as set forth in any one of SEQ ID NO:111, SEQ ID NO: 2, SEQ ID NO: 10, SEQ ID NO: 11, SEQ ID NO: 33, SEQ ID NO: 34, and SEQ ID NO: 13.

In the present application, the isolated antigen-binding protein may comprise a heavy-chain variable region VH and a light-chain variable region VL. The VH may comprise an amino acid sequence as set forth in SEQ ID NO: 54, and the VL may comprise an amino acid sequence as set forth in SEQ ID NO: 63.

In some cases, the isolated antigen-binding protein may comprise a heavy-chain variable region VH and a light-chain variable region VL. The VH may comprise an amino acid sequence as set forth in any one of SEQ ID NO:110, SEQ ID NO: 9, SEQ ID NO: 12, and SEQ ID NO: 14, and the VL may comprise an amino acid sequence as set forth in any one of SEQ ID NO:111, SEQ ID NO: 10, SEQ ID NO: 11, and SEQ ID NO: 13.

For example, the isolated antigen-binding protein may comprise a heavy-chain variable region VH and a light-chain variable region VL. The VH may comprise an amino acid sequence as set forth in SEQ ID NO: 9, and the VL may comprise an amino acid sequence as set forth in SEQ ID NO: 10. For example, the isolated antigen-binding protein may comprise a heavy-chain variable region VH and a light-chain variable region VL. The VH may comprise an amino acid sequence as set forth in SEQ ID NO: 9, and the VL may comprise an amino acid sequence as set forth in SEQ ID NO: 11. For example, the isolated antigen-binding protein may comprise a heavy-chain variable region VH and a light-chain variable region VL. The VH may comprise an amino acid sequence as set forth in SEQ ID NO: 12, and the VL may comprise an amino acid sequence as set forth in SEQ ID NO: 13. For example, the isolated antigen-binding protein may comprise a heavy-chain variable region VH and a light-chain variable region VL. The VH may comprise an amino acid sequence as set forth in SEQ ID NO: 14, and the VL may comprise an amino acid sequence as set forth in SEQ ID NO: 11. For example, the isolated antigen-binding protein may comprise a heavy-chain variable region VH and a light-chain variable region VL. The VH may comprise an amino acid sequence as set forth in SEQ ID NO: 1, and the VL may comprise an amino acid sequence as set forth in SEQ ID NO: 2. For example, the isolated antigen-binding protein may comprise a heavy-chain variable region VH and a light-chain variable region VL. The VH may comprise an amino acid sequence as set forth in SEQ ID NO: 32, and the VL may comprise an amino acid sequence as set forth in SEQ ID NO: 34. For example, the isolated antigen-binding protein may comprise a heavy-chain variable region VH and a light-chain variable region VL. The VH may comprise an amino acid sequence as set forth in SEQ ID NO: 32, and the VL may comprise an amino acid sequence as set forth in SEQ ID NO: 33. The antigen-binding protein of the present application may further comprise a heavy-chain constant region and a light-chain constant region. The heavy-chain constant region may be derived from a human IgG1 constant region, and the light-chain constant region may be derived from a human κ light-chain constant region.

For example, the isolated antigen-binding protein may comprise a heavy-chain variable region VH and a light-chain variable region VL; the VH may comprise an amino acid sequence as set forth in SEQ ID NO: 9; and the VL may comprise an amino acid sequence as set forth in SEQ ID NO: 10. Furthermore, the isolated antigen-binding protein may comprise a heavy-chain constant region and a light-chain constant region; the heavy-chain constant region may comprise an amino acid sequence as set forth in SEQ ID NO: 29; and the light-chain constant region may comprise an amino acid sequence as set forth in SEQ ID NO: 31. The isolated antigen-binding protein may be called HB0030.

For example, the isolated antigen-binding protein may comprise a heavy-chain variable region VH and a light-chain variable region VL; the VH may comprise an amino acid sequence as set forth in SEQ ID NO: 9; and the VL may comprise an amino acid sequence as set forth in SEQ ID NO: 11. Furthermore, the isolated antigen-binding protein may comprise a heavy-chain constant region and a light-chain constant region; the heavy-chain constant region may comprise an amino acid sequence as set forth in SEQ ID NO: 29; and the light-chain constant region may comprise an amino acid sequence as set forth in SEQ ID NO: 31. The isolated antigen-binding protein may be called HB0031.

For example, the isolated antigen-binding protein may comprise a heavy-chain variable region VH and a light-chain variable region VL; the VH may comprise an amino acid sequence as set forth in SEQ ID NO: 12; and the VL may comprise an amino acid sequence as set forth in SEQ ID NO: 13. Furthermore, the isolated antigen-binding protein may comprise a heavy-chain constant region and a light-chain constant region; the heavy-chain constant region may comprise an amino acid sequence as set forth in SEQ ID NO: 29; and the light-chain constant region may comprise an amino acid sequence as set forth in SEQ ID NO: 31. The isolated antigen-binding protein may be called HB0032.

For example, the isolated antigen-binding protein may comprise a heavy-chain variable region VH and a light-chain variable region VL; the VH may comprise an amino acid sequence as set forth in SEQ ID NO: 14; and the VL may comprise an amino acid sequence as set forth in SEQ ID NO: 11. Furthermore, the isolated antigen-binding protein may comprise a heavy-chain constant region and a light-chain constant region; the heavy-chain constant region may comprise an amino acid sequence as set forth in SEQ ID NO: 29; and the light-chain constant region may comprise an amino acid sequence as set forth in SEQ ID NO: 31. The isolated antigen-binding protein may be called HB0033.

For example, the isolated antigen-binding protein may comprise a heavy-chain variable region VH and a light-chain variable region VL; the VH may comprise an amino acid sequence as set forth in SEQ ID NO: 1; and the VL may comprise an amino acid sequence as set forth in SEQ ID NO: 2. Furthermore, the isolated antigen-binding protein may comprise a heavy-chain constant region and a light-chain constant region; the heavy-chain constant region may comprise an amino acid sequence as set forth in SEQ ID NO: 30; and the light-chain constant region may comprise an amino acid sequence as set forth in SEQ ID NO: 31. The isolated antigen-binding protein may be called 900424.

For example, the isolated antigen-binding protein may comprise a heavy-chain variable region VH and a light-chain variable region VL; the VH may comprise an amino acid sequence as set forth in SEQ ID NO: 32; and the VL may comprise an amino acid sequence as set forth in SEQ ID NO: 34. Furthermore, the isolated antigen-binding protein may comprise a heavy-chain constant region and a light-chain constant region; the heavy-chain constant region may comprise an amino acid sequence as set forth in SEQ ID NO: 30; and the light-chain constant region may comprise an amino acid sequence as set forth in SEQ ID NO: 31. The isolated antigen-binding protein may be called 900423.

For example, the isolated antigen-binding protein may comprise a heavy-chain variable region VH and a light-chain variable region VL; the VH may comprise an amino acid sequence as set forth in SEQ ID NO: 32; and the VL may comprise an amino acid sequence as set forth in SEQ ID NO: 33. Furthermore, the isolated antigen-binding protein may comprise a heavy-chain constant region and a light-chain constant region; the heavy-chain constant region may comprise an amino acid sequence as set forth in SEQ ID NO: 30; and the light-chain constant region may comprise an amino acid sequence as set forth in SEQ ID NO: 31. The isolated antigen-binding protein may be called 900428.

The isolated antigen-binding protein of the present application can compete with a reference antibody to bind to the TIGIT protein, wherein the reference antibody may comprise heavy-chain variable regions and light-chain variable regions; the heavy-chain variable regions of the reference antibody may comprise HCDR1, HCDR2 and HCDR3; the HCDR1 may comprise an amino acid sequence as set forth in SEQ ID NO:3; the HCDR2 may comprise an amino acid sequence as set forth in SEQ ID NO:56; the HCDR3 may comprise an amino acid sequence as set forth in SEQ ID NO:57; the light-chain variable regions of the reference antibody may comprise LCDR1, LCDR2 and LCDR3; the LCDR1 may comprise an amino acid sequence as set forth in SEQ ID NO:65; the LCDR2 may comprise an amino acid sequence as set forth in SEQ ID NO:7; and the LCDR3 may comprise an amino acid sequence as set forth in SEQ ID NO:66.

The isolated antigen-binding protein of the present application can compete with a reference antibody to bind to the TIGIT protein, wherein the reference antibody may comprise heavy-chain variable regions and light-chain variable regions; the heavy-chain variable regions of the reference antibody may comprise HCDR1, HCDR2 and HCDR3; the HCDR1 may comprise an amino acid sequence as set forth in SEQ ID NO:3; the HCDR2 may comprise an amino acid sequence as set forth in SEQ ID NO:4; the HCDR3 may comprise an amino acid sequence as set forth in SEQ ID NO:5; the light-chain variable regions of the reference antibody may comprise LCDR1, LCDR2 and LCDR3; the LCDR1 may comprise an amino acid sequence as set forth in SEQ ID NO:6; the LCDR2 may comprise an amino acid sequence as set forth in SEQ ID NO:7; and the LCDR3 may comprise an amino acid sequence as set forth in SEQ ID NO:8.

The protein, polypeptide, and/or amino acid sequence involved in the present application should also be understood to cover at least the following range: a variant or homologue that has the same or similar functions as the protein or polypeptide.

In the present application, the variant may be a protein or polypeptide formed by substituting, deleting or adding one or more amino acids in an amino acid sequence of the defined protein and/or polypeptide (for example, the antigen-binding protein of the present application). For example, the functional variant may comprise a protein or polypeptide with amino acid changes induced by substituting, deleting, and/or inserting at least one amino acid, for example, 1-30, 1-20, or 1-10 amino acids, and for another example, 1, 2, 3, 4, or 5 amino acids. The functional variant may substantially maintain the biological properties of the protein or polypeptide before the changes (for example, substitution, deletion, or addition). For example, the functional variant may maintain at least 60%, 70%, 80%, 90%, or 100% of the biological activity (for example, the capability of binding to an antigen) of the defined protein or polypeptide before the changes. For example, the substitution may be conservative substitution.

In the present application, a moiety of the amino acid sequence of the antigen-binding protein may be homologous to the corresponding amino acid sequence in an antibody from a specific species, or belong to a specific class. For example, both the variable and constant moieties of an antibody may be derived from the variable and constant regions of an antibody of one animal species (such as human). In the present application, the homologue may be a protein or polypeptide that has at least about 85% (for example, at least about 85%, about 90%, about 91%, about 92%, about 93%, about 94%, about 95%, about 96%, about 97%, about 98%, about 99% or higher) sequence homology with the amino acid sequence of the defined protein and/or polypeptide (for example, the antigen-binding protein of the present application).

In the present application, the homology generally refers to the similarity, resemblance or association between two or more sequences. The "percentage of sequence homology" may be calculated in the following way: comparing two sequences to be aligned in a comparison window, determining in the two sequences the number of positions at which identical nucleic acid bases (for example, A, T, C, G) or identical amino acid residues (for example, Ala, Pro, Ser, Thr, Gly, Val, Leu, Ile, Phe, Tyr, Trp, Lys, Arg, His, Asp, Glu, Asn, Gln, Cys, and Met) exist to acquire the number of matching positions; and dividing the number of matching positions by the total number of positions in the comparison window (i.e., the window size), and multiplying a result by 100 to produce the sequence homology percentage. The alignment for determining the sequence homology percentage may be achieved in a variety of ways known in the art, for example, by using publicly available computer software such as BLAST, BLAST-2, ALIGN or Megalign (DNASTAR) software. Those skilled in the art may determine appropriate parameters for sequence alignment, including any algorithm required to implement the maximum alignment undergoing comparison, within a full-length sequence range or within a target sequence region. The homology may also be determined by the following methods: FASTA and BLAST. For the description of the FASTA algorithm, a reference may be made to W. R. Pearson and D. J. Lipman, "Improved Tools for Biological Sequence Comparison", Proc. Natl. Acad. Sci.), 85: 2444-2448, 1988; and D. J. Lipman and W. R. Pearson, "Rapid and Sensitive Protein Similarity Searches", Science, 227: 1435-1441, 1989. For the description of the BLAST algorithm, a reference may be made to S. Altschul, W. Gish, W. Miller, E. W. Myers and D. Lipman, "Basic Local Alignment Search Tool", J. Mol. Biol., 215: 403-410, 1990.

The antigen-binding protein (for example, the TIGIT antibody) of the present application can specifically bind to the TIGIT antigen. The antigen-binding protein (for example, an antibody) that "specifically binds" to the TIGIT antigen can generally bind to the TIGIT at a KD value of about 1 nM or higher (for example, 1 nM, 100 pM, 10 pM, 2 pM, or 1 pM), but not bind to other proteins that lack a TIGIT sequence. For example, the antibody that "specifically binds" to the TIGIT does not bind to human CD226, human CD155, and human CD112. The antigen-binding protein (for example, the antibody) of the present application can specifically bind to the TIGIT antigen or a labeled form thereof (for example, a fluorescently-labeled TIGIT antigen), but not bind to other proteins that lack a TIGIT epitope. Whether the antigen-binding protein (for example, the antibody) binds to the TIGIT antigen may be determined using any assay known in the art. Examples of the assays known in the art for determining the binding affinity comprise surface plasmon resonance (for example, BIACORE) or similar techniques (for example, KinExa or OCTET). In some cases, the TIGIT antibody of the present application may also cross-react with a monkey and/or mouse TIGIT. For example, it may be detected by flow cytometry and enzyme-linked immunosorbent assay. As used herein, "cross-reactivity" refers to the capability of an antibody to react with homologous proteins from other species. The antigen-binding protein (for example, the TIGIT antibody) of the present application can inhibit the binding of TIGIT to a CD155 ligand. A competition method may be used in the blocking experiment for detection. For example, the antigen-binding protein (for example, the TIGIT antibody) is blended with the antigen (or cells capable of expressing the antigen) and the ligand thereof (or, cells expressing the ligand). According to the intensity (for example, fluorescence intensity or concentration) of a detectable label, the ability of the antigen-binding protein to compete with the ligand of the antigen for binding to the antigen is reflected. For example, as detected using the flow cytometer, the IC50 at which the antigen-binding protein (for example, the TIGIT antibody) of the present application blocks the TIGIT antigen from binding to the CD155 ligand is about 0.1 µg/ml-0.05 µg/ml.

### Polyspecific antibody

In another aspect, the present application provides a polyspecific antibody which may comprise a first targeting moiety capable of specifically binding to a TIGIT protein. In the present application, the first targeting moiety of the polyspecific antibody may comprise the isolated antigen-binding protein described above in the present application.

In some cases, the polyspecific antibody may further comprise a second targeting moiety. For example, the second targeting moiety is capable of specifically binding to a tumor-associated antigen. For another example, the second targeting moiety is capable of blocking an interaction between PD-L1 and PD-1. For another example, the second targeting moiety is capable of specifically binding to a PD-L1 protein. For another example, the second targeting moiety capable of binding to the PD-L1 protein comprises an antibody or an antigen-binding fragment thereof. For another example, the antigen-binding fragment capable of binding to the PD-L1 protein comprises Fab, Fab', an Fv fragment, F(ab')2, scFv, di-scFv and/or dAb. For another example, the antibody capable of binding to the PD-L1 protein is selected from the group consisting of a monoclonal antibody, a chimeric antibody, a humanized antibody, and a fully human antibody.

In the present application, the second targeting moiety of the polyspecific antibody may comprise an antibody capable of binding to the PD-L1 protein or an antigen-binding fragment thereof.

In the present application, the antibody capable of binding to the PD-L1 protein may comprise HCDR3. For example, the HCDR3 may comprise an amino acid sequence as set forth in SEQ ID NO: 102.

In the present application, the antibody capable of binding to the PD-L1 protein may comprise HCDR2. For example, the HCDR2 may comprise an amino acid sequence as set forth in SEQ ID NO: 101.

In the present application, the antibody capable of binding to the PD-L1 protein may comprise HCDR1. For example, the HCDR1 may comprise an amino acid sequence as set forth in SEQ ID NO: 100.

In the present application, the antibody capable of binding to the PD-L1 protein may comprise HCDR1, HCDR2, and HCDR3. For example, the HCDR1, HCDR2, and HCDR3 may comprise amino acid sequences as set forth in SEQ ID NO: 100, 101, and 102, respectively.

In the present application, the antibody capable of binding to the PD-L1 protein may comprise VH. For example, the VH may comprise an amino acid sequence as set forth in SEQ ID NO: 106 or 108. In the present application, the antibody capable of binding to the PD-L1 protein may comprise LCDR3. For example, the LCDR3 may comprise an amino acid sequence as set forth in SEQ ID NO: 105.

In the present application, the antibody capable of binding to the PD-L1 protein may comprise LCDR2. For example, the LCDR2 may comprise an amino acid sequence as set forth in SEQ ID NO: 104.

In the present application, the antibody capable of binding to the PD-L1 protein may comprise LCDR1. For example, the LCDR1 may comprise an amino acid sequence as set forth in SEQ ID NO: 103.

In the present application, the antibody capable of binding to the PD-L1 protein may comprise LCDR1, LCDR2, and LCDR3. For example, the LCDR1, LCDR2, and LCDR3 may comprise amino acid sequences as set forth in SEQ ID NO: 103, 104, and 105, respectively.

In the present application, the antibody capable of binding to the PD-L1 protein may comprise VL. For example, the VL may comprise an amino acid sequence as set forth in SEQ ID NO: 107 or 109. In the present application, the polyspecific antibody may comprise a first polypeptide chain and a second polypeptide chain.

In the present application, the first polypeptide chain may comprise the heavy-chain variable region VH of the antibody capable of binding to the PD-L1 protein, the heavy-chain variable region VH capable of binding to the TIGIT protein, and the light-chain variable region VL capable of binding to the TIGIT protein; and the second polypeptide chain may comprise the light-chain variable region VL of the antibody capable of binding to the PD-L1 protein.

In some cases, in the first polypeptide chain, the VH of the antibody capable of binding to the TIGIT protein and the VL of the antibody capable of binding to the TIGIT protein may form scFv. In some cases, the VH of the antibody capable of binding to the TIGIT protein and the VL of the antibody capable of binding to the TIGIT protein may form scFv by a linker peptide. For example, the linker peptide may comprise an amino acid sequence as set forth in any one of SEQ ID NOs: 74 to 77. For example, the sequence of the scFv capable of binding to the TIGIT protein may comprise an amino acid sequence as set forth in any one of SEQ ID NOs: 97 to 99.

In some cases, in the first polypeptide chain, the scFV capable of binding to the TIGIT protein may be located at an N-terminus or C-terminus of the VH of the antibody capable of binding to the PD-L1 protein.

For example, in the first polypeptide chain, the N-terminus of the VL of the antibody capable of binding to the TIGIT protein may be optionally linked to the C-terminus of the VH of the antibody capable of binding to the PD-L1 protein by a linker peptide, and the C-terminus of the VL capable of binding to the TIGIT protein may be optionally linked to the N-terminus of the VH of the antibody capable of binding to the TIGIT protein by a linker peptide.

For another example, in the first polypeptide chain, the N-terminus of the VH capable of binding to the TIGIT protein may be optionally linked to the C-terminus of the VH of the antibody capable of binding to the PD-L1 protein by a linker peptide, and the C-terminus of the VH capable of binding to the TIGIT protein may be optionally linked to the N-terminus of the VL of the antibody capable of binding to the TIGIT protein by a linker peptide.

In the present application, the first polypeptide chain may further comprise a constant region, for example, a human IgG constant region, and the human IgG constant region may be located at the C-terminus of the VH of the antibody capable of binding to the PD-L1 protein and is located at the N-terminus of the scFv of the VL of the antibody capable of binding to the TIGIT protein. In the present application, the human IgG constant region may be located at the C-terminus of the VH of the antibody capable of binding to the PD-L1 protein and is located at the N-terminus of the VL of the VL of the antibody capable of binding to the TIGIT protein.

In some cases, the human IgG constant region may be linked to the N-terminus of the VL of the antibody capable of binding to the TIGIT protein directly or indirectly, for example, by a linker peptide. In some other cases, the human IgG constant region may be located at the C-terminus of the VH of the antibody capable of binding to the PD-L1 protein and is located at the N-terminus of the VH of the antibody capable of binding to the TIGIT protein. For example, the constant region may comprise an amino acid sequence as set forth in SEQ ID NO.: 29 or 30.

In some cases, the human IgG constant region may be linked to the N-terminus of the VH of the antibody capable of binding to the TIGIT protein directly or indirectly, for example, by a linker peptide. For example, the linker peptide may comprise an amino acid sequence as set forth in any one of SEQ ID NOs: 74 to 77.

For example, the first polypeptide chain may comprise an amino acid sequence as set forth in any one of SEQ ID NOs.: 85 to 87.

In the present application, the first polypeptide chain may comprise the heavy-chain variable region VH of the antibody capable of binding to the PD-L1 protein; and the second polypeptide chain may comprise the light-chain variable region VL of the antibody capable of binding to the PD-L1 protein, the heavy-chain variable region VH capable of binding to the TIGIT protein, and the light-chain variable region VL capable of binding to the TIGIT protein.

In some cases, in the second polypeptide chain, the VH of the antibody capable of binding to the TIGIT protein and the VL of the antibody capable of binding to the TIGIT protein may form scFv. In some cases, the VH of the antibody capable of binding to the TIGIT protein and the VL of the antibody capable of binding to the TIGIT protein may form scFv by a linker peptide.

In some cases, in the second polypeptide chain, the scFV capable of binding to the TIGIT protein is located at an N-terminus or C-terminus of the VL of the antibody capable of binding to the PD-L1 protein.

In the present application, the first polypeptide chain comprises the heavy-chain variable region VH of the antibody capable of binding to the TIGIT protein, the heavy-chain variable region VH capable of binding to the PD-L1 protein, and the light-chain variable region VL capable of binding to the PD-L1 protein; and the second polypeptide chain comprises the light-chain variable region VL of the antibody capable of binding to the TIGIT protein.

In some cases, in the first polypeptide chain, the VH of the antibody capable of binding to the PD-L1 protein and the VL of the antibody capable of binding to the PD-L1 protein form scFv. In some cases, the VH of the antibody capable of binding to the PD-L1 protein and the VL of the antibody capable of binding to the PD-L1 protein form scFv by a linker peptide. For example, the linker peptide may comprise an amino acid sequence as set forth in any one of SEQ ID NOs: 74 to 77. For example, the sequence of the scFv capable of binding to the PD-L1 protein may comprise an amino acid sequence as set forth in any one of SEQ ID NOs: 89 to 96.

In some cases, in the first polypeptide chain, the scFV capable of binding to the PD-L1 protein may be located at an N-terminus or C-terminus of the VH of the antibody capable of binding to the TIGIT protein.

For example, in the first polypeptide chain, the N-terminus of the VL capable of binding to the PD-L1 protein is optionally linked to the C-terminus of the VH of the antibody capable of binding to the TIGIT protein by a linker peptide, and the C-terminus of the VL capable of binding to the PD-L1 protein is optionally linked to the N-terminus of the VH of the antibody capable of binding to the PD-L1 protein by a linker peptide.

For another example, in the first polypeptide chain, the N-terminus of the VH capable of binding to the PD-L1 protein may be optionally linked to the C-terminus of the VH of the antibody capable of binding to the TIGIT protein by a linker peptide, and the C-terminus of the VH capable of binding to the PD-L1 protein may be optionally linked to the N-terminus of the VL of the antibody capable of binding to the PD-L1 protein.

In the present application, the first polypeptide chain may further comprise a human IgG constant region, and the human IgG constant region may be located at the C-terminus of the VH of the antibody capable of binding to the TIGIT protein and is located at the N-terminus of the scFv of the VL of the antibody capable of binding to the TIGIT protein. In the present application, the human IgG constant region may be located at the C-terminus of the VH of the antibody capable of binding to the TIGIT protein and is located at the N-terminus of the VL of the VL of the antibody capable of binding to the PD-L1 protein. For example, the constant region may comprise an amino acid sequence as set forth in SEQ ID NO.: 29 or 30.

In some cases, the human IgG constant region may be linked to the N-terminus of the VL of the antibody capable of binding to the PD-L1 protein directly or indirectly, for example, by a linker peptide. In some other cases, the human IgG constant region may be located at the C-terminus of the VH of the antibody capable of binding to the TIGIT protein and is located at the N-terminus of the VH of the antibody capable of binding to the PD-L1 protein.

In some cases, the human IgG constant region may be linked to the N-terminus of the VH of the antibody capable of binding to the PD-L1 protein directly or indirectly, for example, by a linker peptide. For example, the linker peptide may comprise an amino acid sequence as set forth in any one of SEQ ID NOs: 74 to 77.

For example, the first polypeptide chain may comprise an amino acid sequence as set forth in any one of SEQ ID NOs.: 78 to 83.

In the present application, the first polypeptide chain may comprise the heavy-chain variable region VH of the antibody capable of binding to the TIGIT protein; and the second polypeptide chain may comprise the light-chain variable region VL of the antibody capable of binding to the TIGIT protein, the heavy-chain variable region VH capable of binding to the PD-L1 protein, and the light-chain variable region VL capable of binding to the PD-L1 protein.

In some cases, in the second polypeptide chain, the VH of the antibody capable of binding to the PD-L1 protein and the VL of the antibody capable of binding to the PD-L1 protein may form scFv. In some cases, the VH of the antibody capable of binding to the PD-L1 protein and the VL of the antibody capable of binding to the PD-L1 protein may form scFv by a linker peptide.

In some cases, in the second polypeptide chain, the scFV capable of binding to the PD-L1 protein may be located at an N-terminus or C-terminus of the VL of the antibody capable of binding to the TIGIT protein.

In the present application, the VH of the antibody capable of binding to the TIGIT protein (or PD-L1 protein) and the VL of the antibody capable of binding to the TIGIT protein (or PD-L1 protein) may be modified to facilitate the formation of scFv. For example, amino acid mutations may occur, but do not affect the specific binding and binding affinity of the antibody. For example, amino acid mutations may be carried out to form disulfide bonds, allowing the structure of scFv to become more stable. The amino acid mutations may be those common in the art, for example, amino acid mutations in the framework region, and for another example, the mutation of a 100-position amino acid Q in the light-chain variable region into C, or the mutation of a 44-position amino acid R or G in the heavy-chain variable region into C. These pre-mutation and post-mutation VH and VL are also within the scope of protection of the present application.

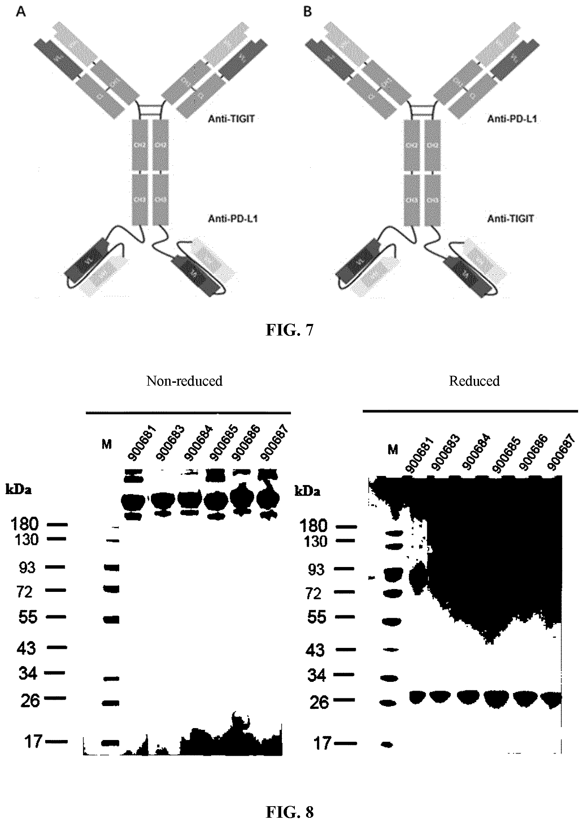

For example, in the present application, the polyspecific antibody may comprise a structure as shown in FIG. 7A, wherein the first polypeptide chain may comprise an amino acid sequence as set forth in any one of SEQ ID NOs: 78 to 83; and the second polypeptide chain may comprise an amino acid sequence as set forth in SEQ ID NO: 84.

For example, in the present application, the polyspecific antibody may comprise a structure as shown in FIG. 7B, wherein the first polypeptide chain may comprise an amino acid sequence as set forth in any one of SEQ ID NOs: 85 to 87; and the second polypeptide chain may comprise an amino acid sequence as set forth in SEQ ID NO: 88.

### Nucleic Acid, vector and cell

In another aspect, the present application further provides an isolated nucleic acid molecule or isolated nucleic acid molecules. The nucleic acid molecule or nucleic acid molecules may encode the polyspecific antibody of the present application. For example, each of the nucleic acid molecule or nucleic acid molecules may encode the reduced polyspecific antibody, or a moiety thereof (for example, one or more of the first targeting moiety, the second targeting moiety, HCDR1-3, LCDR1-3, VL, VH, light chain, or heavy chain).

The nucleic acid molecule(s) of the present application may be isolated. For example, it can be produced or synthesized by the following methods: (i) *in vitro* amplification, for example by polymerase chain reaction (PCR) amplification, (ii) clonal recombination, (iii) purification, for example, by fractionation through restriction digestion and gel electrophoresis, or (iv) synthesis, for example, by chemical synthesis. In some embodiments, the isolated nucleic acid(s) is/are a nucleic acid molecule(s) prepared by the recombinant DNA technology.

In the present application, the nucleic acid encoding the polyspecific antibody may be prepared by a variety of methods known in the art. These methods include, but are not limited to, the overlap extension PCR using restriction fragment operations or using synthetic oligonucleotides. For specific operations, see Sambrook et al., Molecular Cloning, A Laboratory Manual, Cold Spring Harbor Laboratory Press, Cold Spring Harbor, N.Y., 1989; and Ausube et al., Current Protocols in Molecular Biology, Greene Publishing and Wiley-Interscience, New York N.Y, 1993.

In another aspect, the present application provides a vector or vectors, each of which comprises the nucleic acid molecule(s) of the present application. Each vector may comprise the nucleic acid molecule or nucleic acid molecules. In addition, the vector may also comprise other genes, for example, a marker gene that is allowed to select this vector in a suitable host cell and under a suitable condition. In addition, the vector may also comprise an expression control element that allows a coding region to be expressed correctly in a suitable host. Such a control element is well known to those skilled in the art, which, for example, may include a promoter, a ribosome binding site, an enhancer, and other control elements that regulate gene transcription or mRNA translation. In some embodiments, the expression control sequence is a regulatable element. A specific structure of the expression control sequence may vary depending on the function of the species or cell type, but generally includes a 5' non-transcribed sequence and 5' and 3' non-translated sequences, for example, a TATA box, a capped sequence, a CAAT sequence, etc., which are involved in transcription and translation initiation, respectively. For example, the 5' non-transcribed expression control sequence may include a promoter region, and the promoter region may include a promoter sequence for functionally linked to the nucleic acid for transcriptional control. The expression control sequence may further comprise an enhancer sequence or an upstream activator sequence. In the present application, suitable promoters may comprise, for example, promoters for SP6, T3, and T7 polymerases, human U6 RNA promoters, CMV promoters, and their artificial hybrid promoters (such as CMV), wherein a moiety of a promoter may be fused with a moiety of a promoter of an additional cellular protein (such as human GAPDH and glyceraldehyde-3-phosphate dehydrogenase) gene, and the promoter may or may not contain additional introns. The nucleic acid molecule(s) of the present application may be operably linked to the expression control element. The vector may comprise, for example, a plasmid, a cosmid, a virus, a bacteriophage, or other vectors commonly used in, for example, genetic engineering. For example, the vector is an expression vector.

In another aspect, the present application provides a host cell, which may comprise the nucleic acid molecule(s) of the present application and/or the vector or vectors of the present application. In some embodiments, each type of or each host cell may comprise one or one type of the nucleic acid molecule or vector of the present application. In some embodiments, each type of or each cell may comprise a plurality of (for example, 2 or more) or a plurality of types of (for example, 2 or more types of) vectors of the present application. For example, the vector of the present application may be introduced into the host cell, for example, an eukaryotic cell, such as a plant-originated cell, a fungal cell, or a yeast cell, etc. The vector of the present application may be introduced into the host cell by methods known in the art, such as electroporation, lipofectine transfection, lipofectamin transfection, etc.

### Preparation method

In another aspect, the present application provides a preparation method for the polyspecific antibody. The method may comprise culturing the host cell of the present application under such a condition that the polyspecific antibody is expressed. For example, an appropriate medium, an appropriate temperature, a culture time and the like may be used, and these methods are understood by those of ordinary skills in the art.

An exemplary method for producing the anti-human TIGIT antibody of the present application is described in Example 1.

A humanized antibody may be selected from any class of immunoglobulins, including IgM, IgD, IgG, IgA, and IgE. In the present application, the antibody is an IgG antibody, and an IgG1 subtype is used. An essential constant domain sequence may be optimized by screening antibodies with the biological assays described in the Examples below, so as to produce the desired biological activity. Similarly, any type of light chain can be used in the compounds and methods herein. Specifically, κ and λ chains or their variants may be used in the compounds and methods of the present application.

An exemplary method for humanizing the anti-human TIGIT antibody of the present application is described in Example 2.

The sequence of the DNA molecule of the polyspecific antibody or the fragment thereof in the present application may be obtained by conventional techniques, such as methods using PCR amplification or genomic library screening and the like. In addition, the coding sequences of the light and heavy chains may also be fused together to form a single-chain antibody.

Once relevant sequences are obtained, they may be obtained on a large scale by recombination. This is generally done by cloning them into vectors, then transferring then into cells, and then isolating the relevant sequences from the proliferated host cell by means of a conventional method.

In addition, the relevant sequences may also be synthesized by using an artificial synthesis method, in particular when a fragment is short. Generally, a fragment with a very long sequence may be obtained by first synthesizing multiple small fragments, and then linking these small fragments. Then, the nucleic acid molecules may be introduced into various existing DNA molecules (or such as vectors) and cells known in the art.

The present application also relates to vectors comprising the aforementioned appropriate nucleic acid molecules and appropriate promoters or control sequences. These vectors may be used to transform appropriate host cells to enable them to express proteins. The host cells may be prokaryotic cells, such as bacterial cells; or lower eukaryotic cells, such as yeast cells; or higher eukaryotic cells, such as mammalian cells. For example, the animal cells may comprise (but are not limited to): CHO-S, CHO-K1, and HEK-293 cells.

The step of transforming the host cells with recombinant DNAs in the present application may be performed using techniques well known in the art. An obtained transformant may be cultured by a conventional method, and it expresses the polypeptide encoded by the nucleic acid molecule(s) of the present application. According to the host cells used, they are cultured in a conventional medium under suitable conditions. Generally, the host cells are cultured and transformed under conditions suitable for the expression of the polyspecific antibody of the present application. Then, the polyspecific antibody of the present application is purified and obtained using conventional immunoglobulin purification steps, such as protein A-Sepharose, hydroxyapatite chromatography, gel electrophoresis, dialysis, ion exchange chromatography, hydrophobic chromatography, molecular sieve chromatography, or affinity chromatography, and other conventional separation and purification means well known to those skilled in the art.

The resulting monoclonal antibody or polyspecific antibody may be identified by a conventional means. For instance, the binding specificity of the monoclonal antibody or polyspecific antibody may be determined by immunoprecipitation or *in vitro* binding assays, such as fluorescence activated cell sorting (FACS), radioimmunoassay (RIA), or enzyme-linked immunosorbent assay (ELISA).

### Pharmaceutical composition

In another aspect, the present application further provides a composition. In some cases, the composition may be a pharmaceutical composition, which comprises the polyspecific antibody of the present application, and a pharmaceutically acceptable carrier. Generally, these substances may be formulated in a non-toxic, inert, and pharmaceutically acceptable aqueous carrier medium. The formulated pharmaceutical composition may be administered by conventional routes.

The pharmaceutical composition of the present application may be directly used to bind to a TIGIT protein molecule or a PD-L1 protein molecule, and thus can be used to prevent and treat TIGIT-related, PD-L1-related or PD-1-related diseases. The pharmaceutical composition of the present application may comprise the antigen-binding protein of the present application at a safe and effective amount and a pharmaceutically acceptable adjuvant (which may comprise a carrier or an excipient). A pharmaceutical preparation should match the mode of administration.

The polyspecific antibody or pharmaceutical composition described herein may be formulated, dosed, and administered in line with good medical practices. The considerations in this case comprise the specific disorder being treated, the specific mammal being treated, the clinical condition of a single patient, the cause of the disorder, the site of agent delivery, the method of administration, the schedule of administration, and other factors known to a medical practitioner.

### Method and Use

In another aspect, the present application further provides use of the polyspecific antibody, the nucleic acid molecule or nucleic acid molecules, the vector, the cell, and/or the pharmaceutical composition in the preparation of a medicament. The medicament is used for preventing, alleviating and/or treating diseases or disorders, for example, TIGIT abnormality-related diseases. In some cases, the TIGIT abnormality-related diseases may be a T-cell dysfunction disorder. The T-cell dysfunction is manifested in the depletion of T cells, and such a disease is treated or delayed or alleviated by strengthening NK cells and activating T cells to enhance the body immunocompetence. For example, the TIGIT-related disease may be a tumor, a cancer, or an infectious disorder. For example, the TIGIT abnormality-related disease may be a CD155-positive or PVR-positive tumor, cancer, immune disease, or infectious disease, including a tumor, a cancer, an immune disease, or an infectious disease, etc. In some cases, the tumor may include a solid tumor or a non-solid tumor. For example, the tumor may be a colon cancer.

The antigen-binding protein of the present application can inhibit the tumor growth and/or the tumor cell proliferation. In some embodiments, the tumor comprises a colon cancer. In some embodiments, the tumor or cancer is a tumor or cancer with abnormal TIGIT expression.

For example, in a mouse colon cancer model, the antigen-binding protein (for example, the TIGIT antibody) of the present application can slow down the tumor growth.

In another aspect, the present application further provides a method for inhibiting the binding of CD155 to TIGIT, comprising administering the polyspecific antibody.

In another aspect, the present application further provides a method for inhibiting the binding of PD-L1 to PD-1, comprising administering the polyspecific antibody.

Not wishing to be bound by any particular theory, the following examples are merely to illustrate the antigen-binding protein, preparation method, uses and the like of the present application, and are not intended to limit the scope of the present application. Experimental methods without specific conditions indicated in the following examples usually follow conventional conditions, for example the conditions described in Sambrook et al., Molecular Cloning: A Laboratory Manual (New York: Cold Spring Harbor Laboratory Press, 1989), or according to conditions recommended by the manufacturers. Unless otherwise specified, percentages and parts are weight percentages and parts by weight.

### Examples

### Example 1 Anti-human TIGIT mouse monoclonal antibody: preparation method for original antibody

### 1.1 Preparation of hybridoma cells for producing murine monoclonal antibodies

The method for preparing the murine monoclonal antibodies was based on the hybridoma preparation technology invented by Kohler and Milstein in 1975 (Nature, 1975, 256: 495-497). First, human TIGITs with mouse Fc tag proteins (ACRO, #TIT-H5253) were emulsified with the Freund's adjuvant, and then 5 mice of each strain of BALB/c, CD1, and C57BL/6 were subcutaneously immunized at multiple sites. After three immunizations, the serum was collected to detect the titer by ELISA, and to detect the binding activity and functional activity by FACS. Spleen cells were removed from the selected optimal mice to fuse with SP2/0 myeloma cells. After HAT screening to select hybridoma polyclonal cells, polyclonal cell strains capable of specifically binding to human TIGIT and blocking the binding of TIGIT-CD155 were screened out by using ELISA and FACS methods, and then monoclonalized; then, monoclonal cell strains for specific binding were screened out again by using the ELISA and FACS, and their binding capability to monkey and mouse TIGITs was tested; the monoclonal antibodies that blocked the binding of TIGIT-CD155 and TIGIT-CD122 was screened by the FACS, and subsequently tested in the cell functional activity; the screened monoclonal cell strains were subjected to affinity (Biacore) screening to finally obtain monoclonal hybridoma cell strains expressing the human TIGIT antibody; and the monoclonal hybridoma cell strains were subjected to sequence analysis. The screening data were listed in Table 1.

**Table 1. Hybridoma screening data**

| | Binding activity | Binding activity | Block CD155 | Block CD122 | Functional activity | Affinity |
|---|---|---|---|---|---|---|
| Clone No. | OD value (human) | OD value (monkey) | MFI value | MFI value | FACS (IL2) | KD (M) |
| B2/31B5 | 1.9770 | 1.337 | 840.5 | 612.5 | 92.77% | 8.80E-11 |
| B2/42G10 | 1.3750 | 1.413 | 1117 | 464.5 | 123.61% | 7.92E-11 |
| B3/1C6 | 2.3360 | 0.7422 | 921.5 | 578.5 | 349.78% | 1.39E-12 |
| B3/20G10 | 2.4000 | 0.6439 | 972 | 521 | 107.61% | 5.95E-12 |
| B3/23A11 | 1.7080 | 0.9931 | 954 | 462 | 138.26% | 1.33E-11 |
| B3/29F6 | 1.7470 | 1.082 | 797.5 | 535 | 116.18% | 1.39E-11 |
| B3/33B2 | 1.9780 | 1.324 | 760 | 516 | 108.19% | 1.93E-14 |
| D1/212A1 | 2.2730 | 1.759 | 756 | 505 | 108.57% | 8.42E-12 |
| D1/216F3 | 0.7060 | 2.102 | 706.5 | 504.5 | 129.32% | 5.13E-11 |
| D1/227B5 | 0.9480 | 1.427 | 845 | 598.5 | 147.02% | 2.82E-11 |
| D1/123C4 | 2.0390 | 1.760 | 899.5 | 594 | 121.82% | 7.40E-11 |
| D1/13C8 | 2.1270 | 2.391 | 672.5 | 533 | 124.56% | 1.18E-11 |
| D1/85G6 | 1.3510 | 1.883 | 636.5 | 552 | 138.26% | 5.25E-11 |
| D2/1B7 | 1.5170 | 1.111 | 1240.5 | 598 | 107.04% | 1.66E-10 |
| D2/6G9 | 1.2750 | 2.296 | 1068.5 | 546.5 | 170.25% | 1.07E-10 |
| D2/13D5 | 1.4270 | 0.9609 | 1192.5 | 594.5 | 109.03% | 1.62E-10 |
| D5/14C4 | 2.0510 | 0.6676 | 848.5 | 560.5 | 132.55% | 7.02E-11 |
| D2/18F10 | 1.5050 | 2.413 | 3055.5 | 576 | 143.78% | 4.64E-10 |
| D2/90D6 | 1.4450 | 1.035 | 812 | 590 | 143.40% | 1.49E-10 |
| D2/109H1 | 1.3240 | 1.101 | 1103.5 | 672 | 85.72% | 1.69E-10 |

After high-throughput screening, the finally obtained monoclonal hybridoma cell strains had high affinity and biological activity, and exhibited the binding activity to both humans and monkeys, and the blocking function against the two ligands CD155 and CD122 of the TIGIT. The hybridoma cell strains in Table 1 above were humanized.

### Example 2 Cloning and humanization of variable region gene sequences of anti-TIGIT antibody

### 2.1 Cloning of antibody variable region genes in hybridoma cells

Based on the principle of TAKARA's 5' RACE technology, the cDNA sequences of the variable regions of the mouse antibody expressed by the hybridoma cell strains were cloned. Briefly, the variable region gene-specific cDNAs of the heavy and light chains were synthesized by using the SMARTer 5'RACE synthesis kit (TAKARA, #634859) following the instructions. The 5'- and 3'-terminuses of the cDNA sequences were modified with PCR primers, which were designed to add appropriate leader sequences to the variable region cDNAs of the heavy and light chains, respectively, such that the resulting PCR products could be cloned, by a seamless cloning method, onto a heavy-chain vector pHB-Fc and a light-chain vector pHB-Cκ which are expressed in the existing recombinant antibody. The expression vector pHB-Fc contained the human IgG1 heavy-chain constant region gene sequence, with L234A and L235A (Eu numbering) mutations of the antibody ADCC knock out (KO) effect on CH₂; and the vector pHB-Cκ contained the human κ light-chain constant region gene sequence. The PCR amplification products of the heavy- and light-chain variable regions were cloned into the expression vectors by means of an In-fusion cloning reagent (TAKARA, #639650), and transformed into *E. coli* DH5α competent cells (Yeastem Biotech, #FYE607-80VL). Monoclonal colonies were picked for Sanger sequencing, and antibody variable region sequences were obtained by analysis. The variable region sequence of the anti-TIGIT antibody expressed by B3/29F6 was as follows:
B3/29F6 VH SEQ ID NO:1 
DVQLQESGPGLVKPSQSLSLTCTVTGYSITSDYAWIWIRQFPGNKVEWMGYITSSGSTSYNPS
LKSRISFTRDTSKNQFFLQLTSVTTEDTATYYCARLDFGNYGGAMDYWGQGTSVTVSS
B3/29F6 VL SEQ ID NO:2 
DIVMTQSHKFMSTSIGDRVSITCKASQHVSTAVAWYQQKPGQSPKLLIYSASYRYTGVPDRF
TGSRSGTDFTFTISSVQAEDLAVYYCQQHYITPYTFGGGTKLEIK
in which the underlined sections were CDRs (as defined by IMGT, the sequences were as follows):

**Table 2. CDR sequence of murine anti-TIGIT antibody**

| Domain | | Sequence | SEQ ID NO: |
|---|---|---|---|
| VH | CDR1 | GYSITSDYA | 3 |
| | CDR2 | ITSSGST | 4 |
| | CDR3 | ARLDFGNYGGAMDY | 5 |
| VL | CDR1' | QHVSTA | 6 |
| | CDR2' | SAS | 7 |
| | CDR3' | QQHYITPYT | 8 |

### 2.2 Construction of IgG1 wild-type chimeric expression vector

The IgG1 wild-type chimeric antibody and the ADCC KO type chimeric antibody shared the same light-chain vector, except for amino acids Nos. 234 and 235 of the heavy chain CH₂ on the used vector containing a human heavy-chain constant region sequence, namely, A234/A235 for the ADCC KO type and L234/L235 for the wild type. The principle of the method for constructing the IgG1 wild-type chimeric heavy-chain expression vector was the same as that of the expression vector construction method described in 2.1. That is, the heavy-chain variable region gene was amplified by PCR using primers containing the leader sequences, and then cloned into a vector containing a human heavy-chain constant region sequence (L234/L235 for CH2) by seamless linkage, whereby the construction of the IgG1 wild-type chimeric expression vector was completed.

### 2.3 Expression of chimeric antibodies

The expression vectors obtained in 2.1 and 2.2 were amplified by means of *Escherichia coli,* and a sufficient amount of plasmids were prepared using an endotoxin-free plasmid extraction kit (Tiangen Biotech (Beijing) Co., Ltd., #DP1 17) for transient transfection to express the chimeric antibody. CHO-S cells (ThermoFisher, #R80007) were used as host cells for expression. The two prepared heavy-chain vectors, respectively with the light-chain vectors, were mixed with polyetherimide (PEI, Polysciences, #24765-1) to form liposome complexes, which were transfected into CHO-S cells and incubated in an incubator for 5-7 days. The cell culture supernatant was collected by centrifugation, and purified by a Protein A affinity chromatographic column to obtain an ADCC KO type human-mouse chimeric antibody (number 900424) and an IgG1 wild-type human-mouse chimeric antibody (Protein NO. 900445).

The VH and VL sequences of the chimeric antibodies obtained by the aforementioned method were as follows:
900423 and 900428 VH SEQ ID NO: 32, DVQLQESGPGLVKPSQSLSLTCTVTGYSITSDYAWNWIRQFPGNRLEWMGYISYSGAPRYNP SLKSRISITRDTSKNQFFLQLSFVTTEDTATYYCASLGTDYYAMDYWGQGTSVIVSS
900424 VH SEQ ID NO: 1
900424 VL SEQ ID NO:2
900423 VL SEQ ID NO:34
900428 VL SEQ ID NO:33

DIVMTQSHKFMFTSVGDRVSITCKASQHVSNAVAWYQQKPGQSPKLLIHSASYRYTGVPDR
FTGRGSGTDFTFTINSVQAEDLAVYYCQQYYSLPWTFGGGTKLEIK

Here, the amino acid sequences of the heavy-chain constant region of 900424, 900423, and 900428 are as set forth in SEQ ID NO: 30. The light-chain constant regions are each a human κ light-chain constant region (SEQ ID NO: 31).

### 2.4 Humanization of murine anti-human TIGIT antibody: preparation method for humanized antibodies

The antibody was humanized using the following method. The variable region sequence of the antibody was aligned with available sequences in the NCBI IgBlast database, and by means of identification and analysis, a human framework region (FR) suitable for constructing CDR-grafted heavy and light chains thereon was finally determined.

During transformation, transformation sites were designed according to the conserved amino acid residues in the FR of the human antibody and the important amino acid residues in the FR of the antibody; the variable regions of the heavy and light chains of the chimeric antibodies were respectively designed for humanized mutations; and humanized point-mutation antibody expression plasmids were amplified and constructed using PCR. The humanized point-mutation antibody expression plasmids were expressed in the CHO-S cells and then purified respectively to obtain humanized antibody proteins. The humanized antibodies were screened in terms of receptor binding capability, functional inhibitory activity, ADCC effect and other indicators by using ELISA, Biacore, flow cytometry and other detection methods, and four humanized anti-TIGIT antibodies with excellent properties were obtained. The VH and VL sequences of the resulting humanized anti-TIGIT antibodies were shown as follows:
900461 and 900464 VH SEQ ID NO: 9 
QVQLQESGPGLVKPSETLSLTCTVSGYSITSDYAWIWIRQPPGKGLEWIGYITSSGSTYYNPSL
KSRVTFSVDTSKNQFSLKLSSVTAADTATYYCARLDFGNYGGAMDYWGQGTLVTVSS
900461 VL SEQ ID NO:10 
DIQMTQSPSSLSASVGDRVTITCRASQHVSTAVNWYQQKPGKAPKLLIYSASYLYSGVPSRF
SGSRSGTDFTLTISSLQPEDFATYYCQQHYITPYTFGQGTKLEIK
900464 and 900476 VH SEQ ID NO: 11 
DIQMTQSPSSLSASVGDRVTITCKASQHVSTALAWYQQKPGKAPKLLIYSASYLQSGVPSRF
SGSRSGTDFTLTISSLQPEDFATYYCQQHYITPYTFGQGTKLEIK
900466 VH SEQ ID NO:12 
QVQLQESGPGLVKPSETLSLTCTVSGYSITSDYAWIWIRQPPGKKLEWIGYITSSGSTYYNPSL
KSRVTFSVDTSKNQFSLKLSSVTAADTATYYCARLDFGNYGGAMDYWGQGTLVTVSS
900466 VL SEQ ID NO:13 
DIQMTQSPSSLSASVGDRVTITCKASQHVSTAVNWYQQKPGKAPKLLIYSASYRQSGVPSRF
SGSGSGTDFTLTISSLQPEDFATYYCQQHYITPYTFGQGTKLEIK
900476VH SEQ ID NO:14

QVQLQESGPGLVKPSETLSLTCTVSGYSITSDYAWIWIRQPPGNKLEWIGYITSSGSTYYNPSL
KSRVTFSVDTSKNQFSLKLSSVTAADTATYYCARLDFGNYGGAMDYWGQGTLVTVSS

Here, the underlined sections were CDRs (defined according to IMGT), and 900461, 900464, 900466, and 900476 were the numbers for the four humanized antibody proteins respectively, with the corresponding item numbers of HB0030, HB0031, HB0032, and HB0033.

Here, the amino acid sequences of the heavy-chain constant regions of HB0030, HB0031, HB0032, and HB0033 are each as set forth in SEQ ID NO:29; and the light-chain constant regions are each a human κ light-chain constant region (SEQ ID NO: 31).

### Example 3 Testing of chimeric antibodies and humanized antibodies

### 3.1 Testing of the binding activity of TIGIT antibodies to cells expressing the human TIGIT

Testing of the binding activity of chimeric antibodies 900324, 900423, 900424, and 900428, humanized antibodies HB0030, HB0031, HB0032, and HB0033, and cells (CHOK1-huTIGIT-2A3, Huabo Biopharm) expressing the human TIGIT.

All chimeric antibodies and humanized antibodies were diluted to 20 µg/ml with 1% BSA in PBS solution (1% BSA/PBS), were added to a 96-well U-shaped plate at 20 µL per well; and a negative control (with 1% BSA/PBS added only) was setup at the same time. The suspension of cells (CHOK1-huTIGIT-2A3, Huabo Biopharm) expressing human TIGIT during the logarithmic growth phase were centrifuged (1000 rpm × 5 min) to discard the culture medium; and the cells were resuspended with 1% BSA/PBS until the density of viable cells was 1 × 10⁶/mL, and were added, at 20 µL (2 × 10⁴ cells) per well, to the 96-well U-shaped plate with the anti-TIGIT antibody to react for 30 min at room temperature. After reaction, the 96-well U-shaped plate was resuspended with 1% BSA/PBS, centrifuged (300 g × 3 min) to discard the supernatant, and washed once in such a way; and PE-goat anti-human-Fc (Jackson ImmunoResearch, #109-115-098) diluted at 1:200 was added to react for 15 min in the dark at room temperature. After reaction, the 96-well U-shaped plate was resuspended with 1% BSA/PBS, centrifuged (300 g × 3 min) to discard the supernatant, washed three times in such a way, and finally resuspended with 100 µL of 1% BSA/PBS for each wall; and the fluorescence intensity of a PE channel was detected with a flow cytometer (BD, #CantoII).

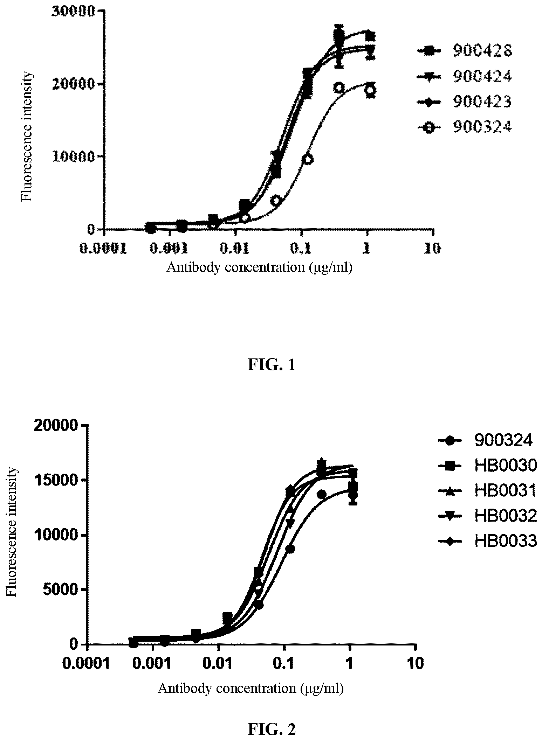

The results of the binding activity of the chimeric antibodies were shown in FIG. 1 and Table 3, and the results of the binding activity of the humanized antibodies were shown in FIG. 2 and Table 4, in which 900324 was the anti-human TIGIT antibody Tiragolumab from Genentech that was transiently expressed in CHO cells, and its light- and heavy-chain variable region sequences were the same as the sequence of the antibody 10A7 in the Patent Application No. WO2015009856A2. The results showed that both the chimeric antibodies and the humanized antibodies exhibited a good and equivalent affinity with the cells (CHOK1-huTIGIT-2A3, Huabo Biopharm) expressing the human TIGIT.

**Table 3. Binding activity of anti-TIGIT chimeric antibodies to human TIGIT**

| Antibody | 900428 | 900424 | 900423 | 900324 |
|---|---|---|---|---|
| EC₅₀(µg/mL) | 0.075 | 0.053 | 0.06 | 0.124 |

**Table 4. Binding activity of anti-TIGIT humanized antibodies to human TIGIT**

| Antibody | 900324 | HB0030 | HB0031 | HB0032 | HB0033 |
|---|---|---|---|---|---|
| EC₅₀₍µg/mL) | 0.08994 | 0.04661 | 0.05806 | 0.07847 | 0.0514 |

### 3.2 Experiment to test TIGIT antibody blocking the binding of TIGIT antigen to TIGIT molecule (competition method)

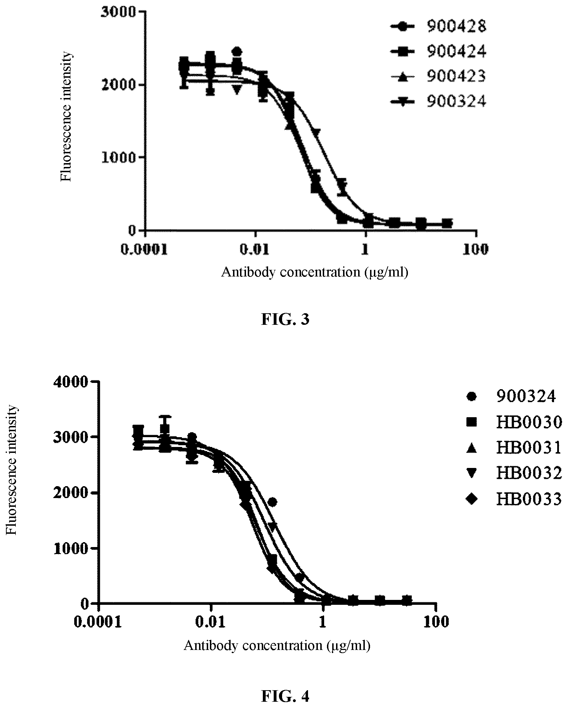

The antigen huCD155-moFc (ACROBiosystems, #CD5-H5254) was diluted with 1% BSA in PBS solution (1% BSA/PBS) to 40 µg/ml, added to a 96-well U-shaped plate at 10 µL per well, and mixed with the serially diluted anti-TIGIT antibody at a volume ratio of 1:1; and a positive control (with CD155-moFc added only) was set up at the same time. The suspension of cells (CHOK1-huTIGIT-2A3, Huabo Biopharm) expressing human TIGIT during the logarithmic growth phase were centrifuged (1000 rpm × 5 min) to discard the culture medium; and the cells were resuspended with 1% BSA/PBS until the density of viable cells was 1 × 10⁶/mL, and were added, at 20 µL (2 × 10⁴ cells) per well, to the 96-well U-shaped plate with the mixture of CD155-moFc and anti-TIGIT antibody to react for 30 min at room temperature. After reaction, the 96-well U-shaped plate was resuspended with 1% BSA/PBS, centrifuged (300 g × 3 min) to discard the supernatant, and washed once in such way; and Alexa488-goat anti-mouse-Fc (Jackson ImmunoResearch, #115-545-071) diluted at 1:300 was added to react for 15 min in the dark at room temperature. After reaction, the 96-well U-shaped plate was resuspended with 1% BSA/PBS, centrifuged (300 g × 3 min) to discard the supernatant, washed three times in such a way, and finally resuspended with 100 µL of 1% BSA/PBS for each wall; and the fluorescence intensity of an FITC channel was detected with a flow cytometer (BD, #CantoII). The results of the binding activity of the chimeric antibodies were shown in FIG. 3 and Table 5, and the results of the binding activity of the humanized antibodes were shown in FIG. 4 and Table 6. The results showed that the chimeric antibodies and the humanized antibodies exhibited a significant and equivalent blocking effect against human CD155.

**Table 5 Blocking activity of anti-TIGIT chimeric antibodies against the binding of human TIGIT to its ligand CD155**

| Antibody | 900428 | 900424 | 900423 | 900324 |
|---|---|---|---|---|
| IC₅₀(µg/mL) | 0.069 | 0.062 | 0.065 | 0.174 |

**Table 6 Blocking activity of anti-TIGIT humanized antibodies against the binding of human TIGIT to its ligand CD155**

| Antibody | 900324 | HB0030 | HB0031 | HB0032 | HB0033 |
|---|---|---|---|---|---|
| IC₅₀(µg/mL) | 0.1349 | 0.05733 | 0.06653 | 0.0919 | 0.05598 |

### 3.3 Affinity test of humanized monoclonal antibody

This test determined the antigen-antibody binding kinetics and affinity by using an SPR method. BIOCORE (GE, #Biacore 8K) was used. A Series S Sensor Chip ProteinA chip (GE, #29-1275-56) was equilibrated at room temperature for 20-30 min, and then loaded into the instrument. An antibody sample was diluted using an equilibration buffer to an experimental working concentration, and sealed at 2-8°C for later use. The antigen, namely, the histidine-tagged (His-Tag) human TIGIT protein (huTIGIT, ACRO Biosystems, #TIT-H52H3), was diluted with an equilibration buffer HBS-EP (10x) (GE, #BR-1006-69). Starting at 20 nM, the antigen was diluted by 2.5-fold for 5 concentration gradients, and 2 replications were set up for zero concentration (i.e., the equilibration buffer) and the lowest concentration. The chip was regenerated with pH1.5 glycine solution (GE, #BR100354). The sample was analyzed by a capture-based multi-cycle kinetics program; the corresponding analysis program was selected to analyze the data, and it was confirmed that there was no obvious reference binding; and Kinetics 1:1 binding model was selected for fitting analysis to acquire the kinetic parameters of the sample.

The testing results were shown in Table 7. The results of the affinity constant (KD(M)) with huTIGIT showed that the affinity of the humanized monoclonal antibody HB0030 of the present application was close to the order of magnitude of 10⁻¹¹, showing a very strong affinity.

**Table 7. Testing results of affinity of humanized Mab-TIGIT antibody with hu TIGIT**

| Antibody | Ka(1/Ms) | Kd(1/s) | KD(M) |
|---|---|---|---|
| HB0030 | 3.17E+06 | 1.85E-04 | 5.83E-11 |

### Example 4 In vivo efficacy test in animals

This experiment studied the efficacy of anti-human TIGIT antibodies HB0030, HB0031, HB0032, and HB0033 at immune checkpoints in humanized mice BALB/c-hPD1/hTIGIT subcutaneously inoculated with the CT26.WT colon cancer tumor model.

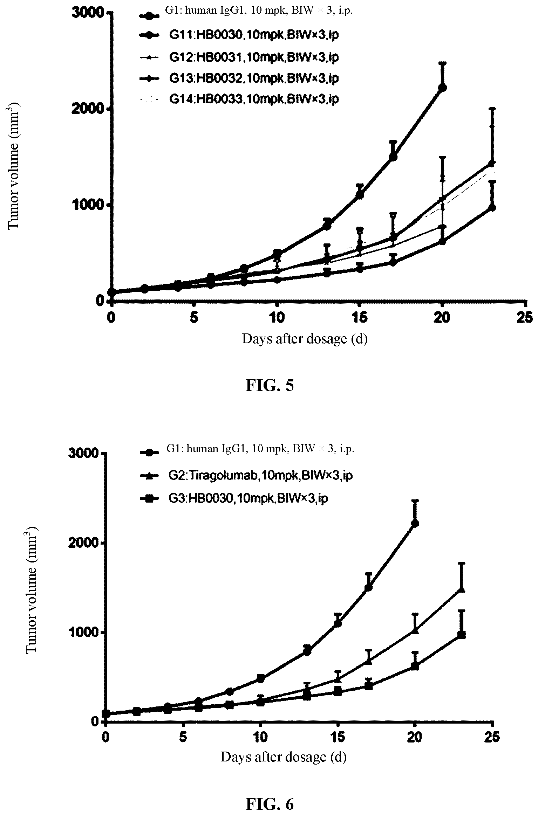

In this experiment, mouse colon cancer cells CT26.WT were used. The CT26.WT cells in the logarithmic growth phase were collected; the culture medium was removed; and the cells were washed twice with PBS, and then inoculated (the survival rate of the CT26.WT cells before and after tumor bearing were 98.57% and 98.28%, respectively), at the inoculation amount of 5 × 10⁵/100 µL/mouse. On the 11th day after inoculation, when the average tumor volume reached 94.02 mm³, the mice were randomized by tumor volume into groups, with 8 mice in each group. The mice were randomized on D0 and dosed on D0, D3, D7, D10, D14, and D17. After the initiation of dosing, the tumor size and body weight of the mice were measured on D0, D2, D4, D6, D8, D10, D13, D15, D17, D20, and D23. The tumor volume was calculated as follows: tumor volume (mm³) = 0.5 × (long tumor diameter × short tumor diameter ²). The results were shown in FIG. 5.

From the experimental results in FIG. 5, it could be observed that the anti-TIGIT humanized monoclonal antibodies HB0030, HB0031, HB0032, and HB0033 of the present application showed an obvious tumor inhibitory effect when administered alone at 10 mg/kg.

A further study was performed on the efficacy of HB0030 and the control antibody Tiragolumab at a dose of 10 mg/kg on the immune checkpoints in the humanized mice BALB/c-hPD1/hTIGIT subcutaneously inoculated with the CT26.WT colon cancer tumor model.

In this experiment, mouse colon cancer cells CT26 were used. The CT26 cells in the logarithmic growth phase were collected; the culture medium was removed; and the cells were washed twice with PBS, and then inoculated (the survival rate of the CT26 cells before and after tumor bearing were 98.51% and 97.250%, respectively), at the inoculation amount of 5 × 10⁵/100 µL/mouse. On the 12th day after inoculation, when the average tumor volume reached 80.33 mm3, the mice were randomly grouped by the tumor volume, with 6 mice in each group. The mice were randomized on D0 and dosed on D0, D3, D7, D10, D14, and D17. After the initiation of dosing, the tumor size and body weight of the mice were measured on D0, D2, D4, D6, D8, D10, D13, D15, D17, D20, and D23. The tumor volume was calculated as follows: tumor volume (mm³) = 0.5 × (long tumor diameter × short tumor diameter ²). The results were shown in FIG. 6.

From the results in FIG. 6, it could be observed that the anti-TIGIT antibody HB0030 of the present application showed a tumor inhibitory effect better than the control antibody.

In this study, on the 24th day after inoculation, the mice in all groups showed no significant change in body weight.

### Example 5 Sequence design of heavy and light chains of bispecific antibodies

The PDL1-TIGIT bispecific antibody was constructed using HB0030, and the structural pattern of this double-antibody was a Morrison mode (IgG-scFv). That is, the C-terminuses of two heavy chains in one IgG antibody were both linked to the scFv fragment of another antibody, with a configuration as shown in FIG. 7A to FIG. 7B. Here, in order to distinguish different antibody forms, when TIGIT was intact IgG and PD-L1 was scFv, the bispecific antibody in such a form was referred to as TIGIT-IgG-PD-L1-scFv, and the main composition design of its heavy chain and light chain was shown in Table 8 below; and when PD-L1 was intact IgG and TIGIT was scFv, the bispecific antibody in such a form was referred to as PD-L1-IgG-TIGIT-scFv, and the main composition design of its heavy and light chains were as shown in Table 9 below.

**Table 8 Main composition design of heavy and light chains of TIGIT-IgG-PD-L1-scFv**

| Bispec ific antibo dy numbe r | Heavy chain | | | Hea vy chai n sequ ence | Light chain variable region | Ligh t chai n sequ ence |
|---|---|---|---|---|---|---|
| | Heavy-chain variable region of IgG moiety | Linker fragm ent | scFv moiety | SEQ ID NO | | SEQ ID NO |
| 90068 1 | HB0030-VH | Linker 2 | (900339-VL-Q100C)-Linker1-(900339-VH-R44C) | 78 | HB0030-VL | 84 |
| 90068 3 | HB0030-VH | Linker 2 | (900339-VL)-Linker2-(900339-VH) | 79 | HB0030-VL | 84 |
| 90068 4 | HB0030-VH | Linker 2 | (900339-VL)-Linker3-(900339-VH) | 80 | HB0030-VL | 84 |
| 90068 5 | HB0030-VH | Linker 2 | (90033 9-VL-Q100C)-Linker3-(900339-VH-R44C) | 81 | HB0030-VL | 84 |
| 90068 6 | HB0030-VH | Linker 2 | (900339-VL)-Linker4-(900339-VH) | 82 | HB0030-VL | 84 |
| 90068 7 | HB0030-VH | Linker 2 | (900339-VL-Q100C)-Linker4-(900339-VH-R44C) | 83 | HB0030-VL | 84 |

**Table 9 Main composition design of heavy and light chains of PD-L1-IgG-TIGIT-scFv**

| Bispeci fic antibod y numbe r | Heavy chain | | | Heavy chain sequen ce | Light chain variable region | Light chain seque nce |
|---|---|---|---|---|---|---|
| | Heavy-chain variable region of IgG moiety | Linker fragme nt | scFv moiety | SEQ ID NO: | | SEQ ID NO: |
| 900691 | 900339-VH | Linker 2 | (HB0030-VL-Q100C)-Linker1-(HB0030-VH-G44C) | 85 | 900339-VL | 88 |
| 900692 | 900339-VH | Linker 2 | (HB0030-VL-Q100C)-Linker2-(HB0030-VH-G44C) | 86 | 900339-VL | 88 |
| 900693 | 900339-VH | Linker 2 | (HB0030-VL-Q100C)-Linker3-(HB0030-VH-G44C) | 87 | 900339-VL | 88 |

In Table 8 and Table 9:
1. the sequence of the antibody 900339 is sourced from a protein 900339 in Patent WO2020199860A1. 900339-VL referred to the light-chain variable region of 900339, and 900339-VH referred to the heavy-chain variable region of 900339. HB0030-VL referred to the light-chain variable region of HB0030, and HB0030-VH referred to the heavy-chain variable region of HB0030.
2. Q100C referred to the mutation of a 100-position amino acid Q in the light-chain variable region into C according to the Kabat antibody numbering scheme; and R44C or G44C referred to the mutation of a 44-position amino acid R or G in the heavy-chain variable region into C according to the Kabat antibody numbering scheme. A disulfide bond was formed after these two amino acid mutations, which enhanced the stability of the scFv region, and increased the druggability of the bispecific antibodies.
3. Amino acid sequence of Linker1: (G4S)3(SEQ ID NO: 74), amino acid sequence of Linker2: (G4S)4(SEQ ID NO: 75), amino acid sequence of Linker3: (G4S)5(SEQ ID NO:76), and amino acid sequence of Linker4: (G4S)6(SEQ ID NO: 77).

### Example 6 Expression and purification of bispecific antibodies

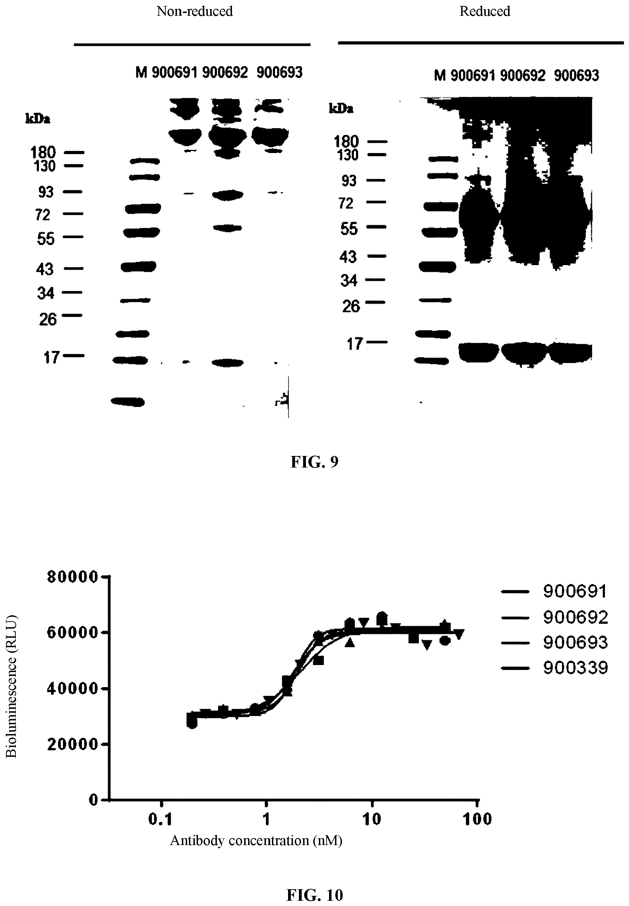

The expression vectors obtained from Example 5 were amplified by means of *Escherichia coli,* and a sufficient amount of plasmids were prepared using an endotoxin-free plasmid extraction kit (Tiangen Biotech (Beijing) Co., Ltd., #DP117) for transient transfection to express the bispecific antibody. CHO-S cells (ThermoFisher, #R80007) were used as host cells for expression. The two prepared heavy-chain vectors, respectively with the light-chain vectors, were mixed with polyetherimide (PEI, Polysciences, #24765-1) to form liposome complexes, which were transfected into CHO-S cells and incubated in an incubator for 5-7 days. The cell culture supernatant was collected through centrifugation, and purified by the Protein A affinity chromatographic column to obtain a series of bispecific antibodies, and the protein aggregation was further determined by the sieve chromatography. The expression, purification and detection of bispecific antibodies were shown in FIG. 8, FIG. 9, and Table 10, and the detection results showed that the SEC purity of 900683 was good for the bispecific antibody in the form of TIGIT-IgG-PD-L1-scFv; and the SEC purity of 900693 was good for the bispecific antibody in the form of PD-L1-IgG-TIGIT-scFv. The reduced and non-reduced SDS-PAGE plots showed that the bispecific antibody in the form of either TIGIT-IgG-PD-L1-scFv or PD-L1-IgG-TIGIT-scFv showed no significant degradation, indicating good protein stability.

**Table 10 SEC purity data of PDL1-TIGIT bispecific antibodies**

| Antibody | 900681 | 900683 | 900684 | 900685 | 900686 | 900687 | 900691 | 900692 | 900693 |
|---|---|---|---|---|---|---|---|---|---|
| SEC Purity (%) | 82.14 | 90.39 | 86.58 | 80.71 | 86.3 | 82.68 | 65.82 | 85.21 | 90.41 |

### Example 7: Determination of kinetic parameters of bispecific antibodies

In this test, the binding kinetics and affinity of the PDL1-TIGIT bispecific antibodies, PDL1 parental monoclonal antibody 900339, and TIGIT parental monoclonal antibody HB0030 to antigens were determined using the SPR method.

The test method was as follows.

The Anti-Human Capture-CM5 chip (GE, #BR-1005-30) was prepared according to the method for conjugating the Human Antibody Capture Kit (GE, #BR-1008-39) to the Amino Conjugation Kit (GE, BR-1000-50). The chip was equilibrated for 20-30 min at room temperature and loaded into the Biacore 8K instrument; the antigens and antibodies were diluted to experimental working concentrations with the equilibration buffer; the antigens were diluted to 50 nM with the equilibrium buffer, and then diluted 3-fold for 7 concentration gradients, and set 2 zero concentrations (i.e., the equilibrium buffer) and one repeat concentration (generally repeating at the minimal concentration); following the order of antibodies, antigens and regeneration, 10 antigen concentrations (2 zero concentrations, 7 gradient concentrations and 1 repeat concentration) were experimentally analyzed in a cyclical manner, and the antigen injection flow rate was 30 µL/min, the binding time was 120 seconds, and the dissociation time was 600 seconds; and after the analysis was completed, the corresponding analysis program was selected to analyze the data, no obvious reference binding was determined, and Kinetics, 1: 1 binding model was selected to fit the data to obtain the kinetics related parameters Ka, Kd and KD values of the bispecific antibodies and the monoclonal-antibody parent antibodies.

The detection results were shown as in Table 11 and Table 12 below, and showed that for the bispecific antibody TIGIT-IgG-PD-L1-scFv, the affinity of the double-antibody to TIGIT was comparable to that of the parent HB0030, and the affinity of the double-antibody to PD-L1 was slightly better than that of the parental monoclonal antibodies 900339. For the bispecific antibody D-L1-IgG-TIGIT-scFv, the affinity of the double-antibodies 900691, 900692, and 900693 to PD-L1 was comparable to that of the parental monoclonal antibody 900339, and the affinity of 900691, 900692 and 900693 to TIGIT was still within the same order of magnitude as the parental monoclonal antibody HB0030.

**Table 11 Detection of affinity of bispecific antibody TIGIT-IgG-PD-L1-scFv to antigen**

| Ligand | Analyte | ka (1/Ms) | kd (1/s) | KD (M) |
|---|---|---|---|---|
| 900339 | Human PD-L 1 | 3.00E+05 | 2.00E-04 | 6.71E-9 |
| 900681 | Human PD-L 1 | 2.90E+05 | 4.27E-04 | 1.47E-9 |
| 900683 | Human PD-L 1 | 4.69E+05 | 4.92E-04 | 1.05E-9 |
| 900684 | Human PD-L 1 | 2.91E+05 | 3.76E-04 | 1.29E-9 |
| 900685 | Human PD-L 1 | 1.91E+06 | 5.27E-04 | 2.76E-9 |
| 900686 | Human PD-L 1 | 4.69E+05 | 4.19E-04 | 8.93E-10 |
| 900687 | Human PD-L 1 | 2.78E+05 | 3.80E-05 | 1.37E-9 |
| HB0030 | Human TIGIT | 5.07E+06 | 1.75E-04 | 3.46E-11 |
| 900681 | Human TIGIT | 3.53E+06 | 2.45E-04 | 6.93E-11 |
| 900683 | Human TIGIT | 4.10E+06 | 1.96E-04 | 4.78E-11 |
| 900684 | Human TIGIT | 3.67E+06 | 1.84E-04 | 5.02E-11 |
| 900685 | Human TIGIT | 4.89E+06 | 2.10E-04 | 4.28E-11 |
| 900686 | Human TIGIT | 4.19E+06 | 2.00E-04 | 4.76E-11 |
| 900687 | Human TIGIT | 3.96E+06 | 2.04E-04 | 5.15E-11 |

**Table 12 Detection of affinity of PD-L1-IgG-TIGIT-scFv bispecific antibody to antigen**

| Ligand | Analyte | ka (1/Ms) | kd (1/s) | KD (M) |
|---|---|---|---|---|
| 900339 | Human PD-L1 | 1.99E+05 | 5.53E-04 | 2.77E-9 |
| 900691 | Human PD-L1 | 2.14E+05 | 4.81E-04 | 2.24E-9 |
| 900692 | Human PD-L1 | 2.04E+05 | 5.70E-04 | 2.79E-9 |
| 900693 | Human PD-L1 | 2.19E+05 | 4.98E-04 | 2.27E-9 |
| HB0030 | Human TIGIT | 4.82E+06 | 1.67E-04 | 3.45E-11 |
| 900691 | Human TIGIT | 9.87E+05 | 9.13E-05 | 9.25E-11 |
| 900692 | Human TIGIT | 1.21E+06 | 9.11E-05 | 7.51E-11 |
| 900693 | Human TIGIT | 9.30E+05 | 8.03E-05 | 8.63E-11 |

### Example 8: Bioactivity determination of bispecific antibodies

### 8.1 Functional inhibition effect of PDL1-TIGIT bispecific antibody against PDL1

The functional inhibition effect of the PDL1-TIGIT bispecific antibody against PDL1 was tested as follows: diluting the bispecific antibodies and the parental monoclonal antibody 900339 were diluted to 10 µg/ml with a PBS solution, and then performing 2-fold dilution for 9 gradients. Target cells Jurkat-NFAT-PD-1-5B8 overexpressing PD-1 were counted, resuspended to 5×10E5/mL, and spread at 30 µl per well into a 96-well white-bottom plate. Effector cells CHO-K1-OS8-PD-L1-8D6 overexpressing PD-L1 were counted, resuspended to 5×10E5/mL, and added at 30 µl per well to the 96-well white-bottom plate in which Jurkat-NFAT-PD-1-5B8 was spread earlier. Then, the diluted bispecific antibodies and the parental control antibodies were sequentially added at 30 µl per well to the 96-well white-bottom plate in which the cells were spread, and incubated for 6 h at 37°C. After the incubation ended, the culture plate was equilibrated for at least 15 min at room temperature, then 90 µL of BIO-Glo assay was added to each well to react for 5 min at room temperature in a dark place, and finally, the values were read by the MDi3x microplate reader to detect the signal intensity.

The detection results of the PD-L1-IgG-TIGIT-scFv bispecific antibody were shown as in FIG. 10 and Table 13, and the results showed that the bispecific antibodies 900691, 900692, and 900693 and the PD-L1 parental monoclonal antibodies 900339 had a comparable functional inhibition effect against PD-L1.

**Table 13 Detection of functional inhibition effect of PD-L1-IgG-TIGIT-scFv bispecific antibody against PDL1 with fluorescent reporter enzyme**

| Antibody | 900339 | 900691 | 900692 | 900693 |
|---|---|---|---|---|
| EC50 (nM) | 1.794 | 1.841 | 2.091 | 1.980 |

### 8.2 Functional inhibition effect of PDL1-TIGIT bispecific antibody against TIGIT

The experimental procedures to detect the functional inhibition effect of the PDL1-TIGIT bispecific antibody against TIGIT were as follows: diluting the bispecific antibodies and the parental monoclonal antibody HB0030 to 10 µg/ml with the PBS solution, and then performing 2-fold dilution for 9 gradients. Target cells Jurkat-TIGIT-22G8 overexpressing TIGIT were counted, resuspended to 4×10E6/mL, and spread to a 96-well white-bottom plate at 50 µl per well. Then, the diluted bispecific antibodies and the parental control antibody were sequentially added at 50 µl per well to the 96-well white-bottom plate in which the cells were spread, and incubated for 10 min at 37°C. Then, 50 µl of PHA at a concentration of 20 µg/ml and 50 µl of CD155 at a concentration of 8 ug/ml were added, mixed well, and incubated for 24 h in an incubator. After the incubation ended, the resultant was centrifuged for 5 min at 1500 rpm to remove the supernatant, and then, the IL-2 content was detected with the ELISA kit.

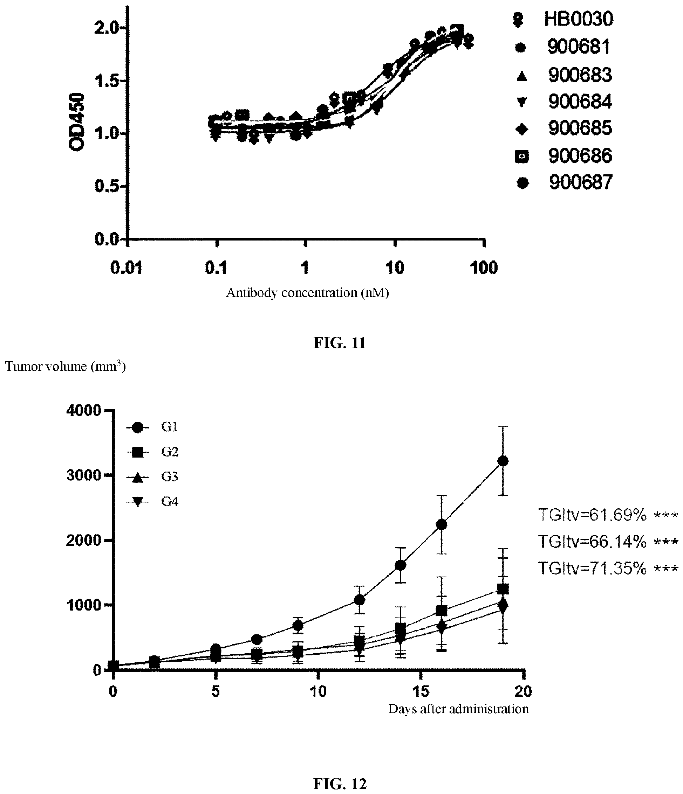

The detection results of the bispecific antibody TIGIT-IgG-PD-L1-scFv were shown as in FIG. 11 and Table 14, and the results showed that no significant difference in the functional inhibition effect against TIGIT was found between the bispecific antibody in the form of TIGIT-IgG-PD-L1-scFv and the TIGIT parent monoclonal antibody HB0030.

**Table 14 IL2 secretion assay to detect the functional inhibition effect of bispecific antibody TIGIT-IgG-PD-L1-scFv against TIGIT**

| Antibody | HB0030 | 900681 | 900683 | 900684 | 900685 | 900686 | 900687 |
|---|---|---|---|---|---|---|---|
| EC50 (nM) | 5.817 | 8.959 | 8.645 | 11.06 | 12.09 | 12.88 | 11.02 |

### Example 9 Detection of thermostability of PDL1-TIGIT bispecific antibody

The experimental procedures for the thermostability of the PDL1-TIGIT bispecific antibody were as follows: detecting the melting temperature (Tm) and aggregation temperature (Tagg) of the bispecific antibodies 900691, 900692, and 900693 in the form of PD-L1-IgG-TIGIT-scFv by a protein stability analyzer (UNcle, UNCHAINED LABS, US), wherein the temperature rise range of 25°C to 95°C and the heating rate of 0.3°C for Tm and Tagg.

The detection results of the thermostability of the bispecific antibodies in the form of PD-L1-IgG-TIGIT-scFv were shown in Table 15 below, and the results showed that the thermostability data of Tm and Tagg values of the bispecific antibodies 900691, 900692, and 900693 in the form of PD-L1-IgG-TIGIT-scFv were in the range of 65°C to 70°C, indicating good thermostability for these three bispecific antibodies.

**Table 15 Thermostability data Tm and Tagg values detected for bispecific antibodies in the form of PD-L1-IgG-TIGIT-scFv**

| Antibody | Tm value (°C) | Tagg value (°C) |
|---|---|---|
| 900691 | 69.2 | 66.5 |
| 900692 | 69.4 | 67.1 |
| 900693 | 70.1 | 67.5 |

### Example 10 Detection of in vivo therapeutic effect of bispecific antibodies against cancers

In this experiment, an animal model of MC38-hPD-L1 colon cancer was established by using B-hPD-1/hPD-L1/hTIGIT humanized mice to test the efficacy of the tested antibodies. Firstly, the animal model of MC38-hPD-L1 colon cancer was established from the B-hPD-1/hPD-L1/hTIGIT humanized mice; the tumor-forming mice were evenly distributed into 7 experimental groups according to the tumor volume and body weight of the mice, with 6 mice in each group, and each group was administrated via intraperitoneal injection, 2 times a week for a total of 6 doses. The specific dosage regimen was shown as in Table 16 below:

**Table 16 Dosage regimen**

| Group | Test article | Number of animals | Route of administr ation | Dosing frequency^{a} | Number of administrat ion |
|---|---|---|---|---|---|
| G1 | 900201 | 6 | i.p. | BIW | 6 |
| G2 | 900339 | 6 | i.p. | BIW | 6 |
| G3 | HB0030 | 6 | i.p. | BIW | 6 |
| G4 | 900339 + HB0030 | 6 | i.p. | BIW | 6 |
| G5 | 900693/900692/900691 low-dose group | 6 | i.p. | BIW | 6 |
| G6 | 900693/900692/900691 medium-dose group | 6 | i.p. | BIW | 6 |
| G7 | 900693/900692/900691 high-dose group | 6 | i.p. | BIW | 6 |

| | | | | | |
|---|---|---|---|---|---|
| Note: 900201 was a IgG1-TNP isotype control; 900339 was a recombinant humanized anti-human PD-L1 monoclonal antibody; HB0030 was a recombinant humanized anti-human TIGIT monoclonal antibody; 900693, 900692 and 900691 were recombinant humanized anti-human PD-L1/TIGIT monoclonal antibody fusion proteins; and a: BIW refers to 2 doses per week. | | | | | |

Measurement of tumor volume and tumor weight: after grouping, the tumor volume was measured twice a week, and at the end of the experiment, the tumor was removed from each mouse and weighed; the tumor volume inhibition rate TGITV was calculated and statistically analyzed; and the tumor inhibitory effect of the test medicament on the model mice was evaluated.

Weight and general clinical observation: the body weight was measured twice a week during the test, and the general clinical observation was performed once a day during the adaptive feeding period and the experimental period to evaluate the safety and tolerability of the test drug to the model mice. The results showed that the bispecific antibody 900693/900692/900691 described in the present application could inhibit the tumor volume and tumor weight growth in the MC38-hPD-L1 colon cancer mice, showing certain safety and tolerability.

### Example 11 Detection of in vivo therapeutic effect of bispecific antibodies against cancers

In this experiment, the in vivo therapeutic effects of the bispecific antibodies against cancers were evaluated by subcutaneously transplanting mouse colorectal cancer cells CT26-hPDL1 to the humanized BALB/C-hPD1/hPDL1/hTIGIT mouse model.

The mouse colorectal cancer cells CT26-hPDL1 were seeded to the right backs of female humanized BALB/C-hPD1/hPDL1/hTIGIT mice. When the tumors of tumor-bearing mouse grew to 70.03 mm3 on average, the mice were randomly divided according to tumor volume into 4 groups (6 mice in each group), including 900201 (a control antibody, 3 mg/kg), 900339 (an hPDL1 antibody, 3 mg/kg), HB0030 (a TIGIT antibody, 3 mg/kg), and 900693 (a bispecific antibody, 4.1 mg/kg) groups. After grouping, the mice in each group were administered intraperitoneally for 3 weeks, twice a week, for a total of 6 times. The tumor volume and body weight were measured weekly, and the relationship between the body weight and tumor volume of the tumor-bearing mice over dosing time was recorded. The results were shown as in FIG. 12 (with G1-G4 sequentially representing the administration results of 900201, 900458, HB0030, and 900693, respectively). The results of FIG. 12 showed that the bispecific antibodies of the present application exhibited significant antitumor effects in the humanized mice BALB/C-hPD1/hPDL1/hTIGIT subcutaneously transplanted with the mouse colorectal cancer cells CT26-hPDL1, and were better than those of 900458 and HB0030, showing potential clinical prospects.

## Claims

1. A polyspecific antibody, comprising a first targeting moiety capable of specifically binding to a TIGIT protein, wherein said first targeting moiety comprises an isolated antigen-binding protein having one or more of the following properties:
1) capability of binding to TIGIT protein at a KD value of 1×10⁻¹⁰ M or lower, wherein said KD value is determined by a surface plasma resonance method;
2) capability of blocking CD155 from binding to TIGIT in a FACS assay; and
3) capability of inhibiting tumor growth and/or tumor cell proliferation.

2. The polyspecific antibody according to claim 1, wherein said antigen-binding protein capable of binding to TIGIT protein comprises at least one CDR in a heavy-chain variable region VH, and said VH comprises an amino acid sequence as set forth in SEQ ID NO: 55.

3. The polyspecific antibody according to any one of claims 1-2, wherein said antigen-binding protein capable of binding to TIGIT protein comprises at least one CDR in a light-chain variable region VL, and said VL comprises an amino acid sequence as set forth in SEQ ID NO: 64.

4. The polyspecific antibody according to any one of claims 1-3, wherein said antigen-binding protein capable of binding to TIGIT protein comprises an antibody or an antigen-binding fragment thereof.

5. The polyspecific antibody according to claim 4, wherein said antigen-binding fragment comprises Fab, Fab', an Fv fragment, F(ab')2, scFv, di-scFv and/or dAb.

6. The polyspecific antibody according to any one of claims 4 to 5, wherein said antibody is selected from the group consisting of: a monoclonal antibody, a chimeric antibody, a humanized antibody, and a fully human antibody.

7. The polyspecific antibody according to any one of claims 1 to 6, wherein said antigen-binding protein capable of binding to TIGIT protein competes with a reference antibody to bind to TIGIT protein, wherein said reference antibody comprises heavy-chain variable regions and light-chain variable regions; said heavy-chain variable regions of said reference antibody comprise HCDR1, HCDR2 and HCDR3; said HCDR1 comprises an amino acid sequence as set forth in SEQ ID NO:3; said HCDR2 comprises an amino acid sequence as set forth in SEQ ID NO: 4 or 42; said HCDR3 comprises an amino acid sequence as set forth in SEQ ID NO: 5 or 43; said light-chain variable regions of said reference antibody comprise LCDR1, LCDR2 and LCDR3; said LCDR1 comprises an amino acid sequence as set forth in SEQ ID NO: 6 or 52; said LCDR2 comprises an amino acid sequence as set forth in SEQ ID NO:7; and said LCDR3 comprises an amino acid sequence as set forth in SEQ ID NO: 8 or 53.

8. The polyspecific antibody according to any one of claims 1-7, wherein said antigen-binding protein capable of binding to TIGIT protein comprises HCDR1, HCDR2 and HCDR3, and wherein said HCDR3 comprises an amino acid sequence as set forth in SEQ ID NO: 57.

9. The polyspecific antibody according to claim 8, wherein said HCDR3 comprises an amino acid sequence as set forth in SEQ ID NO.: 5 or 43.

10. The polyspecific antibody according to any one of claims 8-9, wherein said HCDR2 comprises an amino acid sequence as set forth in SEQ ID NO.: 56.

11. The polyspecific antibody according to any one of claims 8-10, wherein said HCDR2 comprises an amino acid sequence as set forth in SEQ ID NO.: 4 or 42.

12. The polyspecific antibody according to any one of claims 8-11, wherein said HCDR1 comprises an amino acid sequence as set forth in SEQ ID NO.: 3.

13. The polyspecific antibody according to any one of claims 1-12, wherein said antigen-binding protein capable of binding to TIGIT protein comprises framework regions H-FR1, H-FR2, H-FR3, and H-FR4.

14. The polyspecific antibody according to claim 13, wherein the C-terminus of said H-FR1 is directly or indirectly linked to the N-terminus of said HCDR1, and said H-FR1 comprises an amino acid sequence as set forth in SEQ ID NO: 58.

15. The polyspecific antibody according to claim 13 or 14, wherein said H-FR1 comprises an amino acid sequence as set forth in any one of SEQ ID NOs.: 23 and 35.

16. The polyspecific antibody according to any one of claims 13-15, wherein said H-FR2 is located between said HCDR1 and said HCDR2, and said H-FR2 comprises an amino acid sequence as set forth in SEQ ID NO.: 60.

17. The polyspecific antibody according to any one of claims 13-16, wherein said H-FR2 comprises an amino acid sequence as set forth in any one of SEQ ID Nos.: 24, 25, 26, 36, and 37.

18. The polyspecific antibody according to any one of claims 13-17, wherein said H-FR3 is located between said HCDR2 and said HCDR3, and said H-FR3 comprises an amino acid sequence as set forth in SEQ ID NO.: 61.

19. The polyspecific antibody according to any one of claims 13-18, wherein said H-FR3 comprises amino acid sequences as set forth in SEQ ID NOs.: 27, 38, and 39.

20. The polyspecific antibody according to any one of claims 13-19, wherein the N-terminus of said H-FR4 is linked to the C-terminus of said HCDR3, and said H-FR4 comprises an amino acid sequence as set forth in SEQ ID NO.: 62.

21. The polyspecific antibody according to any one of claims 13-20, wherein said H-FR4 comprises amino acid sequences as set forth in SEQ ID Nos.: 28, 40, and 41.

22. The polyspecific antibody according to any one of claims 1-21, wherein said antigen-binding protein capable of binding to TIGIT protein comprises VH, and said VH comprises an amino acid sequence as set forth in SEQ ID NO: 55.

23. The polyspecific antibody according to claim 22, wherein said VH comprises an amino acid sequence as set forth in any one of SEQ ID NOs.: 110, 1, 9, 12, 14, and 32.

24. The polyspecific antibody according to any one of claims 1-23, wherein said antigen-binding protein capable of binding to TIGIT protein comprises an antibody heavy-chain constant region.

25. The polyspecific antibody according to claim 24, wherein said antibody heavy-chain constant region is derived from a human IgG constant region.

26. The polyspecific antibody according to any one of claims 1-25, wherein said antigen-binding protein capable of binding to TIGIT protein comprises LCDR1, LCDR2, and LCDR3, and wherein said LCDR1 comprises an amino acid sequence as set forth in SEQ ID NO: 65.

27. The polyspecific antibody according to claim 26, wherein said LCDR1 comprises an amino acid sequence as set forth in any one of SEQ ID NOs.: 6 and 52.

28. The polyspecific antibody according to claim 26 or 27, wherein said LCDR2 comprises an amino acid sequence as set forth in SEQ ID NO.: 7.

29. The polyspecific antibody according to any one of claims 26-28, wherein said LCDR3 comprises an amino acid sequence as set forth in SEQ ID NO.: 66.

30. The polyspecific antibody according to any one of claims 26-29, wherein said LCDR3 comprises an amino acid sequence as set forth in SEQ ID NO.: 8 or 53.

31. The polyspecific antibody according to any one of claims 1-30, wherein said antigen-binding protein capable of binding to TIGIT protein comprises framework regions L-FR1, L-FR2, L-FR3, and L-FR4.

32. The polyspecific antibody according to claim 31, wherein a C-terminus of said L-FR1 is directly or indirectly linked to the N-terminus of said LCDR1, and said L-FR1 comprises an amino acid sequence as set forth in SEQ ID NO: 68.

33. The polyspecific antibody according to any one of claims 31-32, wherein said L-FR1 comprises an amino acid sequence as set forth in any one of SEQ ID NOs.: 15, 16, 44, and 45.

34. The polyspecific antibody according to any one of claims 31-33, wherein said L-FR2 is located between said LCDR1 and said LCDR2, and said L-FR2 comprises an amino acid sequence as set forth in SEQ ID NO.: 70.

35. The polyspecific antibody according to any one of claims 31-34, wherein said L-FR2 comprises an amino acid sequence as set forth in any one of SEQ ID NOs.: 17, 18, 46, and 47.

36. The polyspecific antibody according to any one of claims 31-35, wherein said L-FR3 is located between said LCDR2 and said LCDR3, and said L-FR3 comprises an amino acid sequence as set forth in SEQ ID NO.: 72.

37. The polyspecific antibody according to any one of claims 31-36, wherein said L-FR3 comprises an amino acid sequence as set forth in any one of SEQ ID NOs.: 19 -21 and 48-50.

38. The polyspecific antibody according to any one of claims 31-37, wherein the N-terminus of said H-FR4 is linked to the C-terminus of said LCDR3, and said L-FR4 comprises an amino acid sequence as set forth in SEQ ID NO.: 73.

39. The polyspecific antibody according to any one of claims 31-38, wherein said L-FR4 comprises an amino acid sequence as set forth in any one of SEQ ID NO.: 22 or 51.

40. The polyspecific antibody according to any one of claims 1-39, wherein said antigen-binding protein capable of binding to TIGIT protein comprises VL, and said VL comprises an amino acid sequence as set forth in SEQ ID NO: 64.

41. The polyspecific antibody according to claim 40, wherein said VL comprises an amino acid sequence as set forth in any one of SEQ ID NOs.: 111, 2, 10, 11, 13, 33, and 34.

42. The polyspecific antibody according to any one of claims 1-41, wherein said antigen-binding protein capable of binding to TIGIT protein comprises an antibody light-chain constant region.

43. The polyspecific antibody according to claim 42, wherein said antibody light-chain constant region is derived from a human κ light-chain constant region.

44. The polyspecific antibody according to any one of claims 1-43, further comprising a second targeting moiety.

45. The polyspecific antibody according to claim 44, wherein said second targeting moiety is capable of specifically binding to a tumor-associated antigen.

46. The polyspecific antibody according to claim 44 or 45, wherein said second targeting moiety is capable of blocking an interaction between PD-L1 and PD-1.

47. The polyspecific antibody according to any one of claims 44-46, wherein said second targeting moiety is capable of specifically binding to a PD-L1 protein.

48. The polyspecific antibody according to claim 47, wherein said second targeting moiety capable of binding to PD-L1 protein comprises an antibody or an antigen-binding fragment thereof.

49. The polyspecific antibody according to claim 48, wherein said antigen-binding fragment capable of binding to PD-L1 protein comprises Fab, Fab', an Fv fragment, F(ab')2, scFv, di-scFv and/or dAb.

50. The polyspecific antibody according to any one of claims 48 49, wherein said antibody capable of binding to said PD-L1 protein is selected from the group consisting of: a monoclonal antibody, a chimeric antibody, a humanized antibody, and a fully human antibody.

51. The polyspecific antibody according to any one of claims 48-50, wherein said antibody capable of binding to PD-L1 protein comprises HCDR1, HCDR2, and HCDR3, and wherein said HCDR3 comprises an amino acid sequence as set forth in SEQ ID NO.: 102.

52. The polyspecific antibody according to claim 51, wherein said HCDR2 comprises an amino acid sequence as set forth in SEQ ID NO.: 101.

53. The polyspecific antibody according to any one of claims 51-52, wherein said HCDR1 comprises an amino acid sequence as set forth in SEQ ID NO.: 100.

54. The polyspecific antibody according to any one of claims 48-53, wherein said antibody capable of binding to PD-L1 protein comprises an heavy-chain variable region VH, and said VH comprises an amino acid sequence as set forth in SEQ ID NO.: 108 or 110.

55. The polyspecific antibody according to any one of claims 48-54, wherein said antibody capable of binding to PD-L1 protein comprises LCDR1, LCDR2, and LCDR3, and wherein said LCDR3 comprises an amino acid sequence as set forth in SEQ ID NO.: 105.

56. The polyspecific antibody according to claim 55, wherein said LCDR2 comprises an amino acid sequence as set forth in SEQ ID NO.: 104.

57. The polyspecific antibody according to any one of claims 55-56, wherein said LCDR1 comprises an amino acid sequence as set forth in SEQ ID NO.: 103.

58. The polyspecific antibody according to any one of claims 48-57, wherein said antibody capable of binding to PD-L1 protein comprises a light-chain variable region VL, and said VL comprises an amino acid sequence as set forth in SEQ ID NO.: 107 or 109.

59. The polyspecific antibody according to any one of claims 1-58, comprising a first polypeptide chain and a second polypeptide chain, wherein said first polypeptide chain comprises a heavy-chain variable region VH of said antibody capable of binding to PD-L1 protein, said heavy-chain variable region VH capable of binding to TIGIT protein, and said light-chain variable region VL capable of binding to TIGIT protein; and said second polypeptide chain comprises said light-chain variable region VL of said antibody capable of binding to said PD-L1 protein.

60. The polyspecific antibody according to claim 59, wherein said first polypeptide chain, said VH of said antibody capable of binding to TIGIT protein and said VL of said antibody capable of binding to TIGIT protein form scFv.

61. The polyspecific antibody according to claim 60, wherein said first polypeptide chain, said scFV capable of binding to TIGIT protein is located at the N-terminus or C-terminus of said VH of said antibody capable of binding to said PD-L1 protein.

62. The polyspecific antibody according to any one of claims 59-61, wherein said first polypeptide chain further comprises a human IgG constant region, and said human IgG constant region is located at said C-terminus of said VH of said antibody capable of binding to said PD-L1 protein and is located at said N-terminus of said VL of said antibody capable of binding to TIGIT protein.

63. The polyspecific antibody according to any one of claims 60-62, wherein said scFv comprises a linker peptide.

64. The polyspecific antibody according to claim 63, wherein said linker peptide comprises an amino acid sequence as set forth in any one of SEQ ID NOs.: 74-77.

65. The polyspecific antibody according to any one of claims 60-64, wherein said scFv has a sequence comprising an amino acid sequence as set forth in any one of SEQ ID NOs.: 97-99.

66. The polyspecific antibody according to any one of claims 59-65, wherein said first polypeptide chain comprises an amino acid sequence as set forth in any one selected from the following group: SEQ ID NOs: 85-87.

67. The polyspecific antibody according to any one of claims 59-66, wherein said second polypeptide chain comprises an amino acid sequence as set forth in SEQ ID NO: 88.

68. The polyspecific antibody according to any one of claims 1-67, comprising a first polypeptide chain and a second polypeptide chain, wherein said first polypeptide chain comprises said heavy-chain variable region VH of said antibody capable of binding to TIGIT protein, said heavy-chain variable region VH capable of binding to PD-L1 protein, and said light-chain variable region VL capable of binding to PD-L1 protein; and said second polypeptide chain comprises said light-chain variable region VL of said antibody capable of binding to TIGIT protein.

69. The polyspecific antibody according to claim 68, wherein in said first polypeptide chain, said VH of said antibody capable of binding to said PD-L1 protein and said VL of said antibody capable of binding to PD-L1 protein form scFv.

70. The polyspecific antibody according to claim 69, wherein said first polypeptide chain, said scFV capable of binding to said PD-L1 protein is located at the N-terminus or C-terminus of said VH of said antibody capable of binding to TIGIT protein.

71. The polyspecific antibody according to any one of claims 68-70, wherein said first polypeptide chain further comprises a human IgG constant region, and said human IgG constant region is located at said C-terminus of said VH of said antibody capable of binding to TIGIT protein and is located at said N-terminus of said VL of said antibody capable of binding to said PD-L1 protein.

72. The polyspecific antibody according to any one of claims 69-71, wherein said scFv comprises a linker peptide.

73. The polyspecific antibody according to claim 72, wherein said linker peptide comprises an amino acid sequence as set forth in any one of SEQ ID NOs.: 74-77.

74. The polyspecific antibody according to any one of claims 69-73, wherein said scFv has a sequence comprising an amino acid sequence as set forth in any one of SEQ ID NOs.: 89-96.

75. The polyspecific antibody according to any one of claims 68-74, wherein said first polypeptide chain comprises an amino acid sequence as set forth in any one selected from the following group: SEQ ID NOs: 78-83.

76. The polyspecific antibody according to any one of claims 59-75, wherein said second polypeptide chain comprises an amino acid sequence as set forth in SEQ ID NO: 84.

77. The polyspecific antibody according to any one of claims 1-76, which is a homodimer.

78. An isolated nucleic acid molecule or isolated nucleic acid molecules, encoding said polyspecific antibody of any one of claims 1-77.

79. A vector, comprising said nucleic acid molecule or nucleic acid molecules according to claim 78.

80. A cell, comprising said nucleic acid molecule or nucleic acid molecules according to claim 78 or said vector according to claim 79.

81. A preparation method for said polyspecific antibody according to any one of claims 1-77, comprising culturing said cell according to claim 80 under such a condition that said polyspecific antibody according to any one of claims 1-77 is expressed.

82. A pharmaceutical composition, comprising said polyspecific antibody of any one of claims 1-77, said nucleic acid molecule or nucleic acid molecules of claim 78, said vector of claim 79, and/or said cell of claim 80, and optionally a pharmaceutically acceptable adjuvant.

83. Use of said polyspecific antibody of any one of claims 1-77, said nucleic acid molecule of claim 78, said vector of claim 79, said cell of claim 80, and/or said pharmaceutical composition of claim 82 in the preparation of a medicament for preventing, relieving and/or treating diseases or disorders.

84. The use according to claim 83, wherein said diseases or disorders comprise a TIGIT-related disease.

85. The use according to any one of claims 83-84, wherein TIGIT-related disease is a T-cell dysfunction disorder.

86. The use according to any one of claims 83-85, wherein said diseases or disorders are tumors.

87. The use according to claim 86, wherein said tumors comprise a colon cancer.

88. A method for preventing, alleviating or treating diseases or disorders, wherein said method comprises administering said polyspecific antibody of any one of claims 1-77 to a subject in need thereof.

89. The method according to claim 88, wherein said diseases or disorders comprise a TIGIT-related disease.

90. The method according to any one of claims 88-89, wherein said TIGIT-related disease is a T-cell dysfunction disorder.

91. The method according to any one of claims 88-90, wherein said diseases or disorders are tumors.

92. The method according to claim 91, wherein said tumors comprise a colon cancer.

93. A method for inhibiting the binding of CD155 to TIGIT, wherein said method comprises administering said polyspecific antibody of any one of claims 1-77.

94. A method for inhibiting the binding of PD-L1 to PD-1, wherein said method comprises administering said polyspecific antibody of any one of claims 46-77.
